(19) 

Europäisches
Patentamt
European
Patent Office
Office européen
des brevets

(11) **EP 2 522 428 A1**

(12) **DEMANDE DE BREVET EUROPEEN**

(43) Date de publication:
**14.11.2012 Bulletin 2012/46**

(51) Int Cl.:
***B01L 7/00*** *(2006.01)*    ***C12Q 1/68*** *(2006.01)*

(21) Numéro de dépôt: **12169223.0**

(22) Date de dépôt: **11.02.2010**

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **11.02.2009 FR 0950860**

(62) Numéro(s) de document de la (des) demande(s) initiale(s) en application de l'article 76 CBE:
**10705839.8 / 2 396 114**

(71) Demandeur: **Institut National de la Recherche Agronomique (INRA)**
**75341 Paris Cedex 07 (FR)**

(72) Inventeurs:
 • **Bodin, Anthony**
  **86100 Chatellerault (FR)**

 • **Priel, Marc**
  **92300 Levallois (FR)**
 • **Fraisse, Pierre-Olivier**
  **94450 Limeil-Brevannes (FR)**
 • **Couture, Carole**
  **33800 Bordeaux (FR)**
 • **Falentin, Cyril**
  **35310 Mordelles (FR)**

(74) Mandataire: **Coralis Harle**
**14-16 Rue Ballu**
**75009 Paris (FR)**

Remarques:
Cette demande a été déposée le 24-05-2012 comme demande divisionnaire de la demande mentionnée sous le code INID 62.

(54) **Procédés pour tester la justesse et la fidélité thermique d'un thermocycleur pour PCR et moyens pour leur mise en oeuvre**

(57) L'invention consiste en un procédé pour tester la justesse et la fidélité thermique d'une enceinte thermiquement contrôlée d'un thermocycleur pour PCR, ladite enceinte thermiquement contrôlée comprenant une pluralité d'emplacements pour tubes de mélange réactionnel, ledit procédé comprenant les étapes suivantes :
a) disposer un ensemble de capteurs de mesure de température dans des emplacements pour tube de mélange réactionnel choisis dans ladite enceinte thermiquement contrôlée, lesdits capteurs de mesure de température comprenant :
(i) un capillaire ou tube adapté aux emplacements prévus dans le carrousel,
(ii) un volume choisi de liquide emplissant au moins partiellement ledit tube réactionnel
(iii) une sonde thermique au moins partiellement immergée dans ledit liquide, et
(iv) au moins un moyen de liaison de ladite sonde thermique à un moyen de réception d'un signal généré par ladite sonde thermique,

lesdits emplacements dans lesquels sont disposés les capteurs de mesure de température étant répartis dans l'enceinte thermiquement contrôlée par air pulsé aux emplacements prévus pour les tubes ou capillaires de PCR de manière qu'au moins la moitié ou qu'au plus 20 de ces emplacements soient occupés par des capteurs de mesure, et
b) démarrer le thermocycleur, dans des conditions normales d'utilisation (c'est-à-dire, couvercle chauffant fermé et actif), pour au moins un cycle programmé avec des valeurs de consigne de température pour une réaction PCR, et
c) simultanément à l'étape b), mesurer les valeurs de température avec chacun des capteurs de mesure, à une pluralité d'instants donnés.

EP 2 522 428 A1

SEQUENCE LISTING

<110>   Institut National de la Recherche Agronomique – INRA

<120>   Procédés pour tester la justesse et la fidélité thermique d'un
        thermocycleur pour PCR, et moyens pour leur mise en oeuvre

<130>   W503PCT

<160>   12

<170>   PatentIn version 3.3

<210>   1
<211>   20
<212>   DNA
<213>   artificial sequence

<220>
<223>   amorce

<400>   1
cacacttcat atttacccat                                                    20


<210>   2
<211>   20
<212>   DNA
<213>   artificial sequence

<220>
<223>   amorce

<400>   2
ttgtttaata gagacgaagg                                                    20


<210>   3
<211>   20
<212>   DNA
<213>   artificial sequence

<220>
<223>   amorce

<400>   3
atggacattt acggtagtgg                                                    20


<210>   4
<211>   20
<212>   DNA
<213>   artificial sequence

<220>
<223>   amorce

<400>   4
aagtatttca atgccggtag                                                    20


<210>   5
<211>   20
<212>   DNA
<213>   artificial sequence

```
<220>
<223>   amorce

<400>   5
gctagctgta actggagccg                                                    20


<210>   6
<211>   20
<212>   DNA
<213>   artificial sequence

<220>
<223>   amorce

<400>   6
gtctgctgaa actgccaaca                                                    20


<210>   7
<211>   20
<212>   DNA
<213>   artificial sequence

<220>
<223>   amorce

<400>   7
ggcctgctga aaatgactga                                                    20


<210>   8
<211>   27
<212>   DNA
<213>   artificial sequence

<220>
<223>   amorce

<400>   8
caaaacaaaa caatatatac attccaa                                            27


<210>   9
<211>   20
<212>   DNA
<213>   artificial sequence

<220>
<223>   amorce

<400>   9
gataaatctg gagccggtga                                                    20


<210>   10
<211>   20
<212>   DNA
<213>   artificial sequence

<220>
<223>   amorce

<400>   10
```

```
gatacgggag ggcttaccat                                                    20


<210>  11
<211>  20
<212>  DNA
<213>  artificial sequence

<220>
<223>  amorce

<400>  11
ccggatcaag agctaccaac                                                    20


<210>  12
<211>  20
<212>  DNA
<213>  artificial sequence

<220>
<223>  amorce

<400>  12
caacccggta agacacgacc                                                    20
```

**Description**

**DOMAINE DE L'INVENTION**

La présente invention se rapporte au domaine de la calibration thermique des appareils thermocycleurs pour la réalisation de réactions PCR, par des moyens physiques ou des moyens biologiques.

**ART ANTERIEUR**

La technique d'amplification d'acides nucléiques par réaction en chaîne à la polymérase (PCR, pour « Polymerase Chain Reaction») est un procédé dont l'utilisation est quasi-incontournable dans le domaine de la génétique moléculaire, y compris dans les domaines aussi divers que le diagnostic médical, l'analyse d'ADN dans le domaine médico-légal, la détection de micro-organismes pathogènes pour l'homme, l'animal ou les plantes, la recherche en biologie moléculaire, etc.

La réaction PCR consiste en une réaction enzymatique *in vitro* thermo-contrôlée d'amplification du nombre de copies d'un acide nucléique présent initialement dans un échantillon. Comme cela est connu, la réaction PCR met en oeuvre un mélange réactionnel comprenant des acides nucléiques amorces et une ADN polymérase thermorésistante, ledit mélange réactionnel étant soumis à des cycles réactionnels multi-étapes thermo-régulés. Classiquement, un cycle de réaction PCR comprend successivement (i) une étape de dénaturation de molécules d'ADN double-brin réalisée à une température spécifique, proche de 95˚C, (ii) une étape d'hybridation des amorces nucléotidiques réalisée à une température spécifique, proche de 60˚C, puis (iii) une étape d'élongation réalisé à une température spécifique, proche de 72˚C.

Les réactions PCR sont réalisées dans des appareils spécifiquement conçus, les thermocycleurs. Les thermocycleurs sont équipés de moyens de régulation précise de la température du mélange réactionnel au cours du temps, à chaque étape (i), (ii) et (ii) ci-dessus, pour chaque cycle d'amplification PCR compris dans le test en cours de réalisation. L'un des éléments principaux d'un thermocycleur consiste en un bloc thermique, constitué de plusieurs éléments thermiquement régulés indépendamment les uns des autres, comprenant une pluralité d'emplacements ou « puits » destinés à recevoir des tubes de mélange réactionnel. Comme cela est connu, un contrôle d'une très grande précision de la température du milieu réactionnel pendant la totalité de la durée d'une réaction PCR est essentiel pour l'obtention de résultats fiables et reproductibles.

Par exemple, une dérive négative de température, par rapport à la température sélectionnée, pendant l'étape de dénaturation peut entraîner une dénaturation incomplète de l'ADN et/ou entraîner une dégradation au moins partielle de l'ADN polymérase thermorésistante.

Egalement, une dérive négative de température pendant l'étape d'hybridation peut entraîner un mésappariement, y compris une hybridation non-spécifique, des amorces nucléotidiques sur l'ADN à amplifier, ce qui est susceptible d'entraîner des résultats faussement positifs. Inversement, une dérive positive de température au moment de l'étape d'élongation peut entrainer une absence d'appariement des amorces avec l'ADN cible, ce qui est susceptible de générer des résultats faussement négatifs.

De plus, une dérive positive ou négative de température au moment de l'étape d'élongation peut entraîner une réduction de l'activité catalytique de l'ADN polymérase de nature à provoquer une réduction du rendement de la réaction PCR.

Comme cela est illustré ci-dessus, la reproductibilité des conditions thermiques dans lesquelles sont effectuées les réactions PCR est essentielle pour la signification biologique des résultats des tests réalisés, et la possibilité de comparer les résultats de tests distincts.

De nombreux auteurs ont observé que la justesse et la fidélité thermique des thermocycleurs PCR (on peut aussi utiliser l'expression « régulation thermique » des thermocycleurs PCR, bien que cette expression soit moins appropriée et moins précise), aussi précis soient-ils, est suffisamment variable pour entraîner un défaut de reproductibilité des résultats obtenus :

(i) pour un même test, sur des thermocycleurs distincts, y compris sur des thermocycleurs distincts du même modèle commercial,
(ii) entre des tests réalisés de manière séparée dans le temps, sur un même thermocycleur, et même
(iii) entre des tests réalisés simultanément dans le temps dans le même thermocycleur, dans des tubes réactionnels disposés de manière géographiquement éloignée à la surface du bloc thermique.

Cette hétérogénéité dans la justesse et la fidélité thermique des mélanges réactionnels utilisés au cours de la réaction PCR, qui est susceptible d'entraîner l'obtention de résultats faussement positifs ou faussement négatifs, selon les types de dérives thermiques, est préoccupante notamment dans le domaine de la réalisation de tests pour le diagnostic médical de maladies.

Afin de surmonter ces inconvénients techniques, des dispositifs de mesure de la justesse et la fidélité thermique des thermocycleurs PCR ont été décrits, capables de détecter des variabilités locales de température dans les puits de réception des tubes réactionnels du bloc thermique. La possibilité de détecter cette variabilité thermique dans des localisations données d'un bloc thermique rend possible une opération de contrôle fin de la justesse et la fidélité thermique du thermocycleur, ou opération de « réglage » thermique du thermocycleur (on peut parfois utiliser aussi le terme « calibrage » thermique du thermocycleur).

Les opérations de réglage thermique des thermocycleurs deviennent dans tous les cas requises pour les laboratoires utilisateurs de thermocycleurs qui sont demandeurs d'accréditations officielles délivrées par des organismes d'Etat.

Un type de dispositif de mesure de la justesse et la fidélité thermique de thermocycleurs PCR connu est le système MTAS® commercialisé par la société Quanta Biotech. Ce système consiste en un support plan, de surface similaire à celle d'un bloc thermique de thermocycleur, sur lequel peuvent être fixées, selon le choix de l'utilisateur, une ou plusieurs sondes thermiques. Le support équipé de la ou des sondes est posé sur la partie supérieure du bloc thermique, la ou les sondes étant alors introduites dans les puits correspondants du bloc thermique. Puis, le thermocycleur est mis en route selon un programme prédéterminé, notamment pour les valeurs de consigne de température aux différentes étapes d'un cycle d'amplification et pour les valeurs de durée de montée ou de descente en température jusqu'aux valeurs de consigne. Simultanément, le signal généré par chacune des sondes thermiques du dispositif MTAS® est transmis à une unité électronique qui enregistre l'évolution des valeurs de température au cours du temps, dans chaque puits du bloc thermique dans lequel est introduite une sonde. Les résultats de mesure permettent de repérer les défauts de régulation thermique du thermocycleur testé et (i) soit simplement prendre ces défauts en considération lors de l'exécution ultérieure de réactions PCR, (ii) soit de procéder à un réglage fin du système de régulation thermique du thermocycleur, c'est-à-dire de procéder à un « réglage » de celui-ci. Un autre système de mesure très similaire est commercialisé sous les noms DRIFTCON® et DRIFTCON® RF par la société éponyme. Ce système diffère du système précédent notamment par une transmission des données générées par les sondes thermiques par voie hertzienne et non par voie filaire.

Un autre système de mesure, basé sur le même principe que celui décrit ci-dessus, est constitué d'une pluralité de sondes thermiques qui peuvent être introduites dans les puits d'une microplaque d'essai d'un type adapté au thermocycleur testé. La microplaque d'essai équipée d'une ou plusieurs sondes thermiques est placée sur le bloc thermique du thermocycleur et l'appareil est mis en fonctionnement selon des cycles prédéfinis par l'utilisateur. Les données de température générées par les sondes sont ensuite enregistrées puis analysées. Un tel système de mesure est par exemple commercialisé sous le nom CYCLERtest® par la société Anachem.

On a également décrit un système d'évaluation de la performance des thermocycleurs pour PCR comprenant une pluralité de tubes contenant un micro-volume d'eau dans lesquels sont introduites des sondes thermiques reliées à un moyen d'acquisition de données (Kim et al., 2008, BioTechniques , Vol. 44 (n°4) : 495-505).

Au moins certains des systèmes de mesure connus paraissent satisfaisants pour pallier l'existence de variabilités thermiques des thermocycleurs par un réglage.

Néanmoins, il existe toujours un besoin pour des systèmes de mesure de la justesse et la fidélité thermique de thermocycleurs PCR, alternatifs ou améliorés par rapport aux systèmes connus.

Ce besoin des systèmes de mesure de la justesse et la fidélité thermique de thermocycleurs PCR existe quel que soit le type de thermocycleur considéré et aussi bien (i) pour les thermocycleurs PCR à plaques équipés avec un bloc thermique comprenant une pluralité d'éléments thermiques que (ii) pour les thermocycleurs PCR à tubes capillaires dans lesquels le chauffage a lieu par la génération d'un courant d'air pulsé chauffé.

## DESCRIPTION DES FIGURES

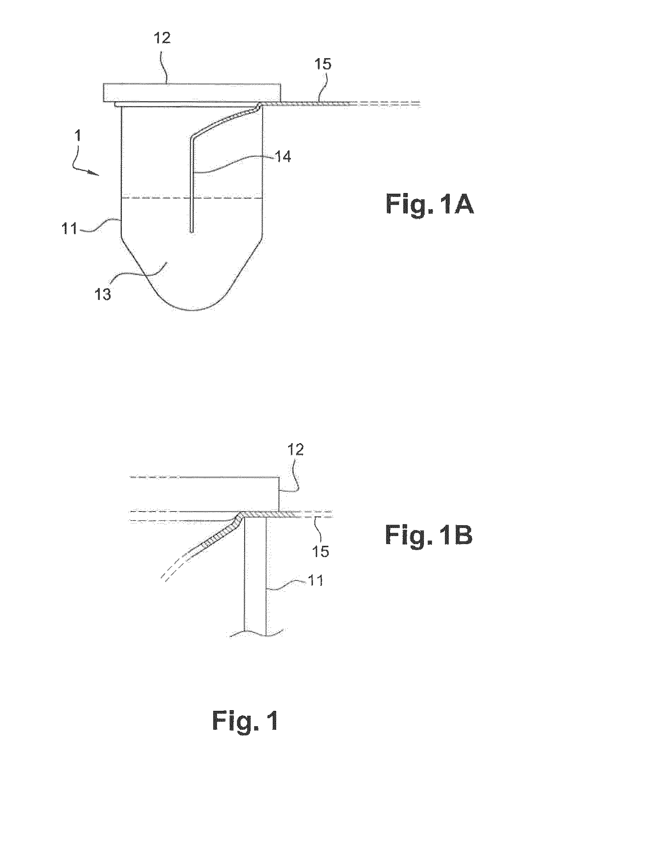

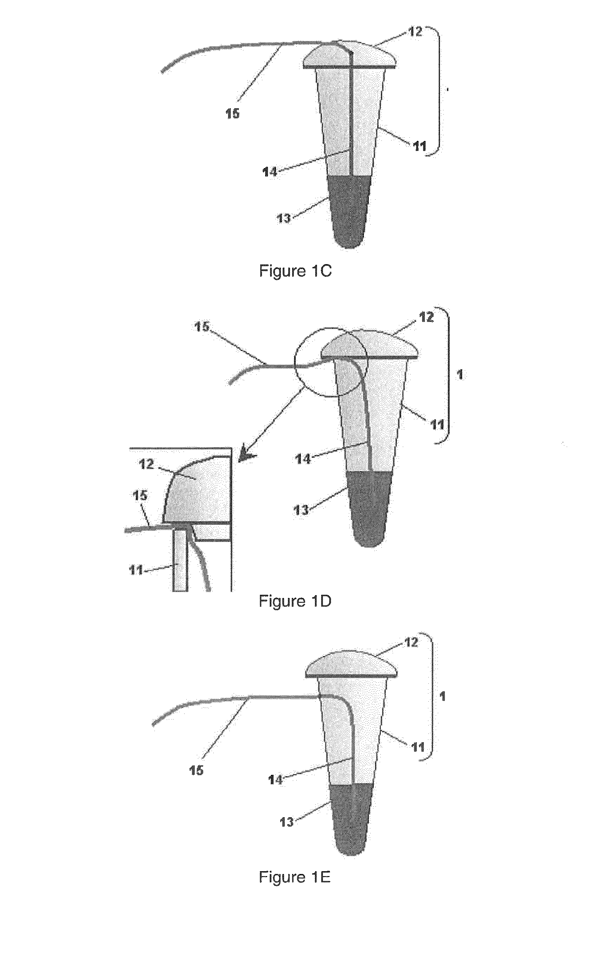

La **Figure 1** est un schéma d'un mode de réalisation particulier d'un capteur de mesure de température utilisé selon l'invention. La Figure 1A représente le capteur et la Figure 1B illustre le passage des moyens de liaison (15) vers l'extérieur du capteur. La Figure 1D est une autre représentation du capteur illustré dans les figures 1A et 1B. La Figure 1C illustre un mode de réalisation du capteur de mesure de température, dans lequel le moyen de liaison de la sonde thermique à un moyen de réception du signal généré par la sonde thermique traverse la paroi du couvercle (12) du tube réactionnel. La Figure 1E illustre un mode de réalisation du capteur de mesure de température, dans lequel le moyen de liaison de la sonde thermique à un moyen de réception du signal généré par la sonde thermique traverse la paroi latérale (11) du tube réactionnel.

La **Figure 2** est un schéma d'une variété de configurations de positionnement des capteurs de mesure sur un bloc thermique de thermocycleur PCR conformément à diverses prescriptions, respectivement.

- figure 2A : issue de la bibliographie (Schoder et al, 2003 et 2005),

- figure 2B : issue d'un rapport d'analyse réalisés par la société Driftcon / Genotronics,
- figure 2C : issue d'une publication interne à l'INRA (Hachet et al, 2005),
- figure 2D : issue d'un rapport d'analyse réalisé par la société Applied Biosystems,
- figure 2E : issue de la norme ISO n° XP CEN ISO/TS 20836 2005,
- figure 2F : issue de l'observation du système commercialisé par la société Aviso
- figure 2G : issue d'une procédure interne antérieure non confidentielle mais non publiée,
- figure 2H : issue de recommandations du Service Après Vente de la société Thermo Electro Corporation

La **Figure 3** est un ensemble de schémas de positionnement des capteurs de mesure sur différents blocs thermiques de thermocycleur pour PCR à plaques de 24 (3A), 30 (3B), 40 (3C), 48 (3D), 60 (3E), 96 (3F, 3G) et 384 (3H, 3I) puits et sur différents dispositifs thermiques de thermocycleur pour PCR à carrousel de 32 (3J), 36 (3K) et 72 (3L) puits.

La **Figure 4** est un schéma d'un système de mesure de la justesse et la fidélité thermique d'un bloc thermique de thermocycleur.

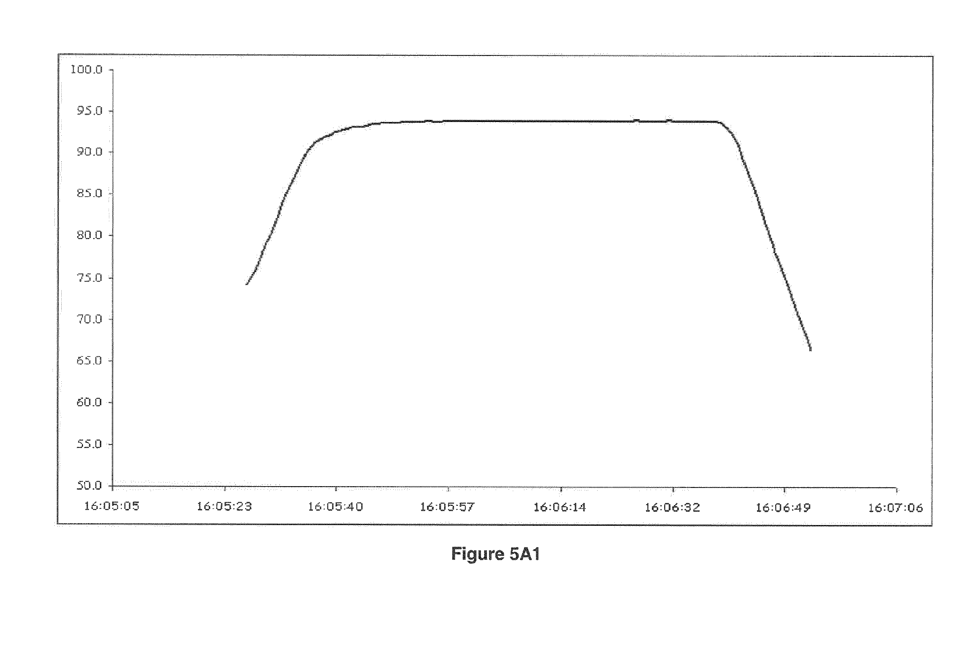

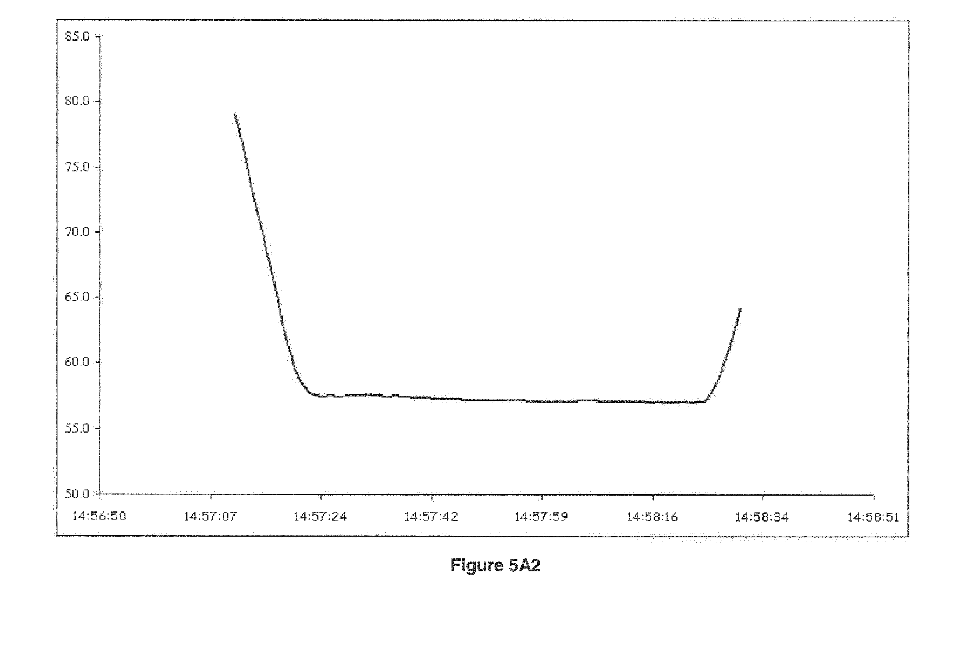

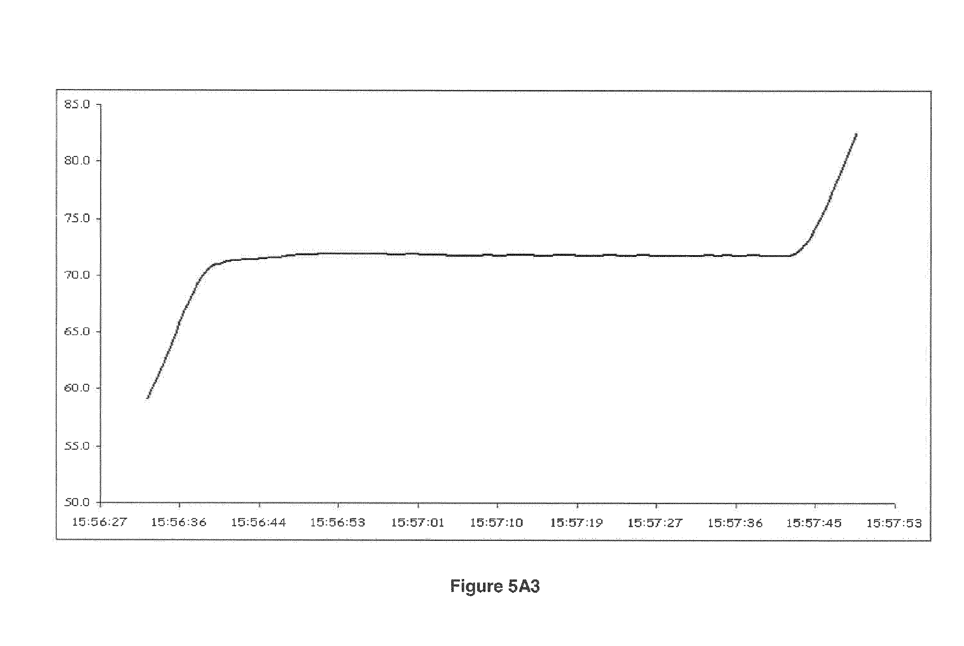

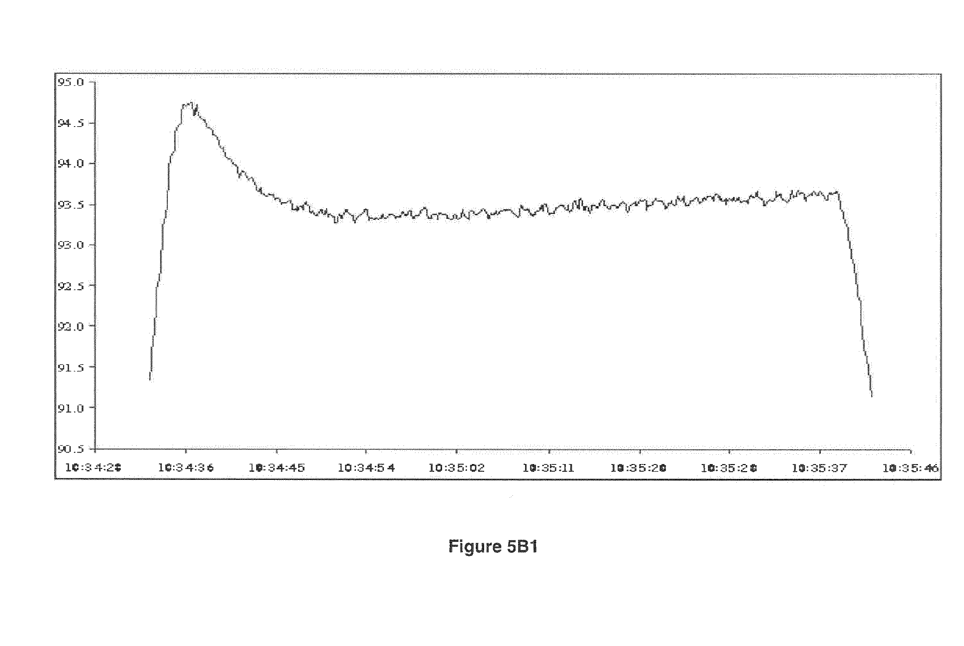

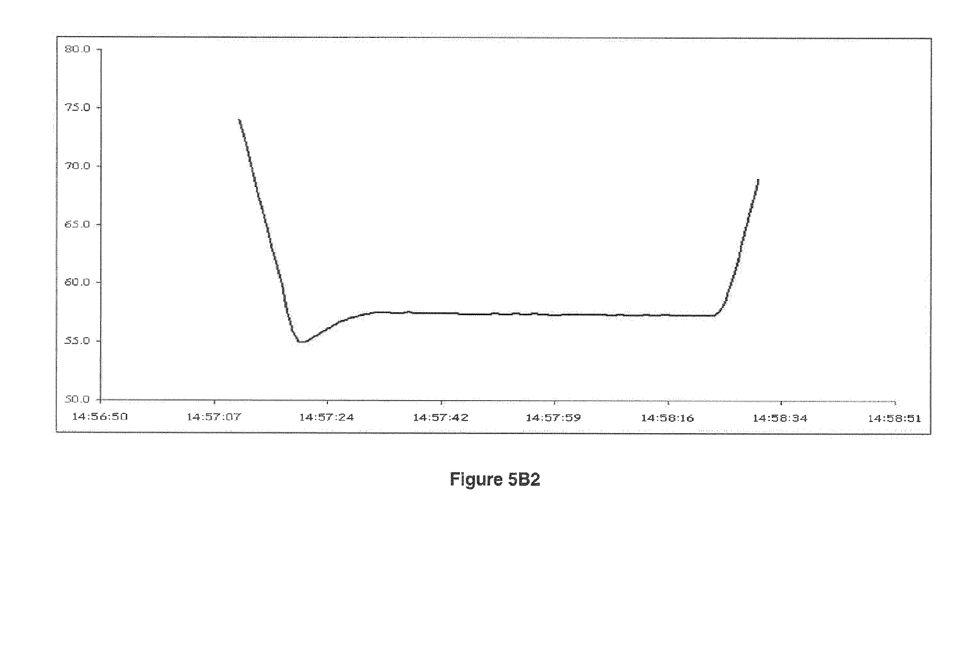

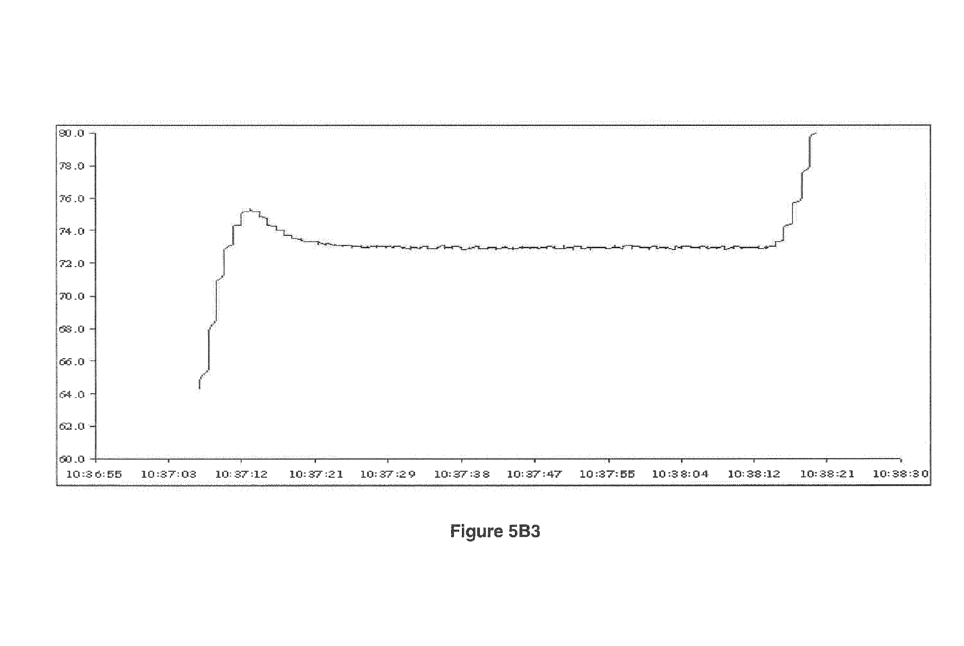

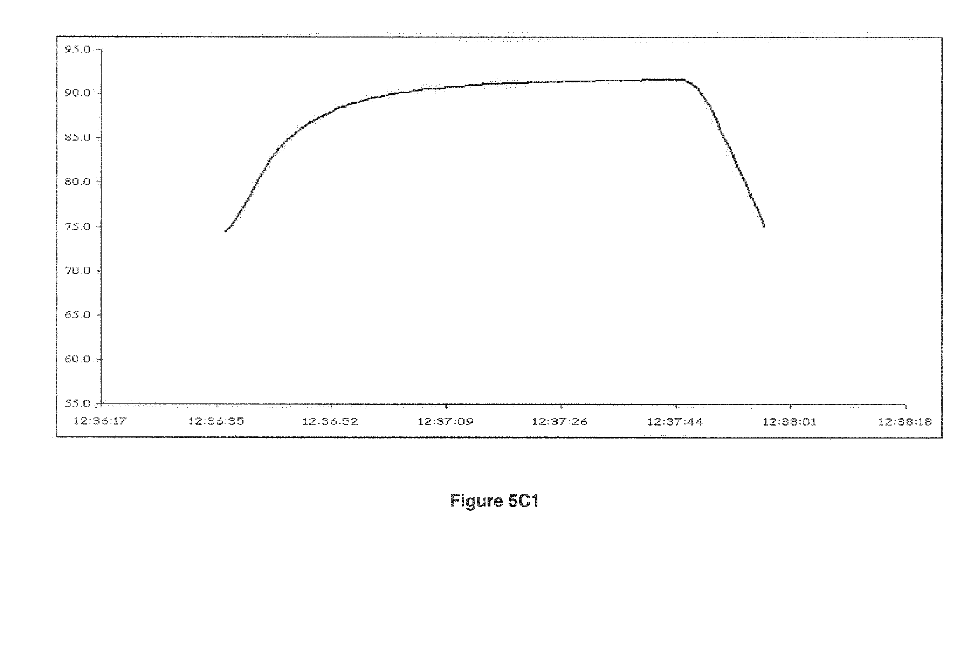

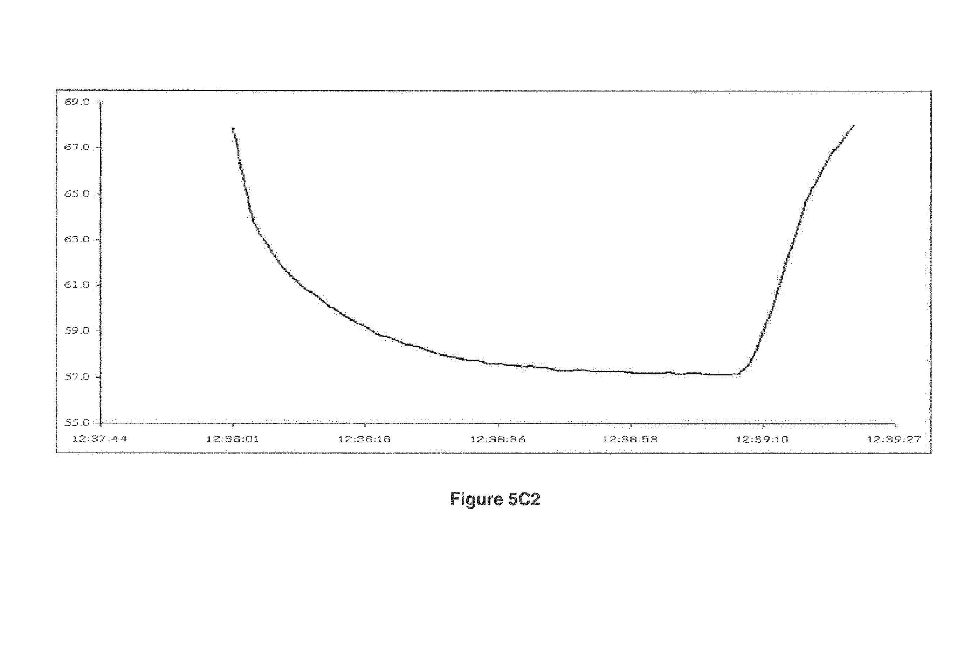

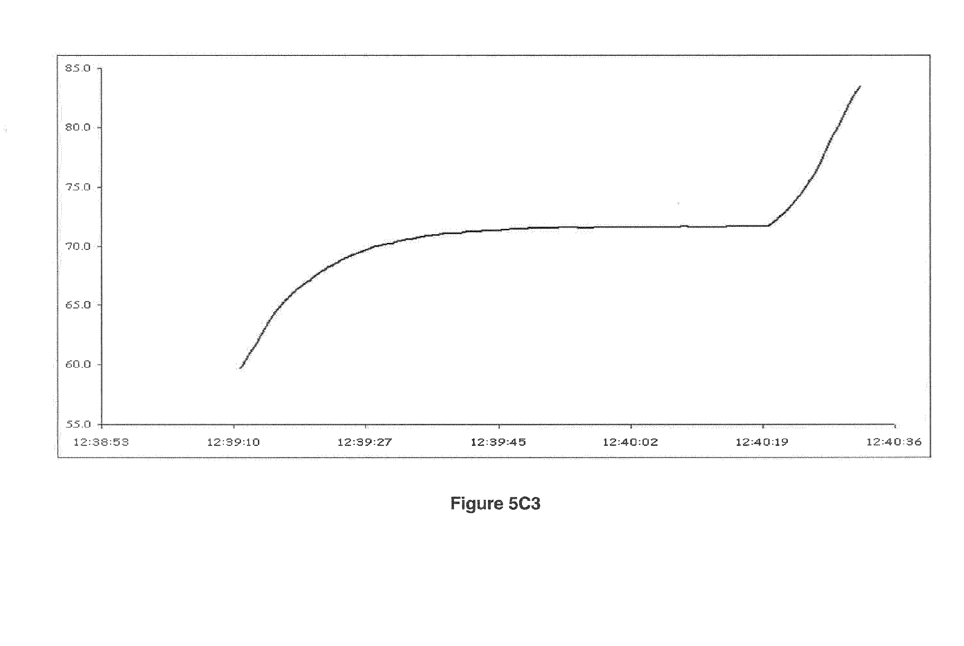

Les **Figures 5A**, **5B** et **5C** illustrent les profils caractéristiques des cinétiques de température pour les étapes de dénaturation, d'hybridation et d'élongation. Les figures 5A1, 5A2 et 5A3 illustrent les profils des cinétiques de températures dans un mode de régulation thermique adapté du thermocycleur. Les figures 5B1, 5B2 et 5B3 illustrent les profils des cinétiques de températures dans un mode de régulation thermique du thermocycleur comportant des excès thermiques (« overshots ») ou des déficits thermiques (« undershots »), avant stabilisation de la température. Les figures 5C1, 5C2 et 5C3 illustrent les profils des cinétiques de températures dans un mode de régulation thermique non linéaire du thermocycleur, c'est-à-dire avec des montées ou des descentes en température trop progressives.

Les **Figures 6 (a, b) et 7 (a, b)** illustrent chacune les résultats d'un test de calibration biologique, respectivement avec un thermocycleur A et avec un thermocycleur B.

Les **Figures 8 (a, b, c)** illustrent chacune les résultats d'un test de calibration biologique, respectivement réalisés avec une température d'hybridation fixe de 57,6°C (8a), 58,3°C (8b), et 59°C (8c), sur les 16 emplacements répartis sur le bloc suivant le schéma 3F ou 3G.

La **Figure 9** illustre les résultats de trois tests de calibration biologique, respectivement à la température seuil Ts moins 1 °C, la température seuil Ts moins 0,5°C et la température seuil Ts.

## DESCRIPTION DETAILLEE DE L'INVENTION

Il est fourni selon l'invention un procédé pour tester la régulation d'un bloc thermique de thermocycleur ou d'une enceinte thermiquement contrôlée par air pulsé pour PCR, dont les caractéristiques détaillées sont spécifiées plus loin dans la présente description.

Le demandeur a observé qu'avec les systèmes connus de mesure de régulation thermique de thermocycleurs PCR, du type comprenant des sondes introduites dans les puits du bloc thermique ou d'une microplaque d'essai, une variabilité dans la régulation du bloc thermique pouvait être efficacement détectée. Toutefois, les variabilités thermiques détectées avec ce type de dispositifs connus reflétaient peu les variations thermiques réelles subies par les mélanges réactionnels. D'une part, il a été observé par le demandeur que ces dispositifs mesuraient des valeurs de température du bloc thermique lui-même et non celles de mélanges réactionnels. D'autre part, le volume important de ces dispositifs interdisait la fermeture du capot du thermocycleur, ce qui entraînait des artéfacts de mesure additionnels de la température, ces artéfacts de mesure additionnels étant très importants lorsque le thermocycleur testé était équipé d'un capot chauffant.

Le demandeur a également observé qu'avec les systèmes connus à tubes du type de ceux décrits par Kim et al. (2008, voir *Supra*), le nombre et la disposition des capteurs de température dans le bloc thermique ne permettait pas de détecter les variabilités thermiques avec suffisamment de précision. En outre, de tels systèmes présentaient l'inconvénient d'être conçus pour un type spécifique de bloc thermique, ce qui signifie qu'autant de types de système que de types de blocs thermiques étaient nécessaires pour procéder au réglage d'une variété d'appareils.

Les inconvénients ci-dessus des systèmes connus ont été résolus selon l'invention.

La présente invention a pour objet un procédé pour tester la justesse et la fidélité thermique d'un appareil thermocycleur pour PCR, ce qui inclut (i) le test de la justesse et la fidélité thermique d'un appareil thermocycleur PCR à plaques, qui est équipé d'un bloc thermique comprenant une pluralité d'éléments thermiquement régulés et (ii) le test de la justesse et la fidélité thermique d'un appareil thermocycleur PCR à tubes capillaires, qui est équipé d'un système de régulation thermique par génération d'air pulsé chauffé.

On précise que les appareils thermocycleurs PCR à air pulsé comprennent une enceinte thermo-régulée dans laquelle sont disposées une pluralité de tubes capillaires (ou tubes plastiques suivant les appareils) dans la lumière desquels a lieu la réaction d'amplification PCR. La régulation thermique de l'enceinte dans laquelle est disposée la pluralité de tubes est réalisée par l'intermédiaire d'un dispositif de génération d'air pulsé, ledit dispositif comprenant au moins un élément chauffant, en général une ou plusieurs résistances électriques. Dans ces appareils thermocycleurs PCR, le courant d'air pulsé, qui peut être généré par un ventilateur disposé dans ledit élément chauffant, entre en contact avec l'élément chauffant et est ainsi chauffé à la température désirée. L'air pulsé et chauffé à la température désirée est expulsé vers l'intérieur de l'enceinte thermorégulée où il entre en contact avec les parois externes de chacun des tubes dans lesquels a lieu la réaction d'amplification PCR. Le mélange réactionnel contenu dans la lumière de chacun des tubes est à son tour porté jusqu'à la température désirée par conduction thermique. Dans ce type d'appareil thermocycleur PCR, les tubes sont en général positionnés selon une disposition circulaire, dans l'enceinte thermorégulée. En général, les tubes sont disposés de manière circulaire au moyen d'un carrousel sur lequel les tubes sont fixés. A titre illustratif, un appareil thermocycleur PCR à tubes capillaires à chauffage par air pulsé peut être par exemple un dispositif commercialisé par la Société Roche sous la dénomination LightCycler®.Egalement à titre illustratif, un appareil thermocycleur PCR à tubes plastiques et à chauffage par air pulsé peut être par exemple un dispositif commercialisé par la Société Qiagen sous la dénomination Rotorgene®.

Dans la présente description, on utilisera de préférence le terme « bloc thermique » pour désigner le moyen de chauffage du mélange réactionnel d'un appareil thermocycleur PCR à plaques.

Dans la présente description, on utilisera de préférence le terme « enceinte thermique » ou « enceinte thermiquement contrôlée » pour désigner le moyen de chauffage du mélange réactionnel d'un appareil thermocycleur PCR à tubes capillaires ou plastiques et à chauffage par air pulsé.

Quel que soit le type d'appareil thermocycleur PCR considéré, respectivement un appareil à plaques ou bien un appareil à tubes capillaires ou plastiques avec chauffage par air pulsé, le conteneur dans lequel est placé le mélange réactionnel et où peut avoir lieu une réaction d'amplification PCR pourra être désigné par le terme commun de « tube de mélange réactionnel ».

La présente invention a pour objet un procédé pour tester la justesse et la fidélité thermique d'un bloc thermique ou d'une enceinte thermiquement contrôlée d'un thermocycleur pour PCR, ledit bloc thermique ou ladite enceinte thermiquement contrôlée comprenant une pluralité d'emplacements pour tubes de mélange réactionnel et une pluralité d'éléments de régulation thermiques, ledit procédé comprenant les étapes suivantes :

a) disposer un ensemble de capteurs de mesure de température dans des emplacements pour tube de mélange réactionnel choisis dans ledit bloc thermique ou ladite enceinte thermiquement contrôlée, lesdits capteurs de mesure de température comprenant :

(i) un tube réactionnel adapté au type de bloc thermique testé,
(ii) un volume choisi de liquide emplissant au moins partiellement ledit tube réactionnel
(iii) une sonde thermique au moins partiellement immergée dans ledit liquide, et
(iv) au moins un moyen de liaison de ladite sonde thermique à un moyen de réception d'un signal généré par ladite sonde thermique,

lesdits emplacements dans lesquels sont disposés les capteurs de mesure de température étant répartis, respectivement :

-    sur la surface dudit bloc thermique de manière qu'au moins deux capteurs de mesure de température sont disposés dans chacun des éléments thermiques compris dans ledit bloc thermique,
-    dans l'enceinte thermiquement contrôlée par air pulsé aux emplacements prévus pour les tubes ou capillaires de PCR de manière qu'au moins la moitié ou qu'au plus 20 de ces emplacements soient occupés par des capteurs de mesure, et

b) démarrer le thermocycleur, dans des conditions normales d'utilisation, pour au moins un cycle programmé avec des valeurs de consigne de température pour une réaction PCR, et
c) simultanément à l'étape b), mesurer les valeurs de température avec chacun des capteurs de mesure, à une pluralité d'instants donnés.

Selon la taille du bloc thermique du thermocycleur à tester, ledit bloc thermique est couramment constitué de deux à huit éléments de régulation thermique contrôlés indépendamment les uns des autres. Le demandeur a montré que le réglage des différents éléments de régulation thermique, au sein d'un même appareil thermocycleur, était imprécis, ce qui générait une variabilité des températures appliquées aux mélanges réactionnels, selon l'élément de régulation thermique dans les puits duquel étaient disposés les tubes réactionnels.

C'est la raison pour laquelle, afin de surmonter cet inconvénient, on dispose, selon le procédé de l'invention, au moins deux capteurs de mesure de température dans les puits de chacun des éléments de régulation thermique constitutifs du bloc thermique du thermocycleur testé, ce qui permet l'obtention d'une mesure plus fiable et reproductible de l'hétérogénéité de régulation thermique dont est susceptible d'être affecté le thermocycleur.

Par « emplacements pour tubes de mélange réactionnel », on entend essentiellement, sinon exclusivement :

- soit les « emplacements » prévus dans une enceinte thermique contrôlée d'un thermocycleur pour PCR, destinés à recevoir des tubes contenant le mélange réactionnel pour réaliser une réaction de PCR,
- soit les « emplacements » moulés en forme de puits pratiqués dans la structure d'un bloc thermique d'un thermocycleur pour PCR, destinés à recevoir les tubes contenant le mélange réactionnel pour réaliser une réaction de PCR , lesdits tubes ayant une géométrie et un volume spécialement adaptés auxdits puits ou emplacements.

Par «capteur de mesure » de température, on entend un capteur qui possède la combinaison de caractéristiques définies ci-dessus. En référence à la figure 1A, qui représente un mode de réalisation d'un capteur thermique, ledit capteur thermique comprend un tube réactionnel (1), ou un capillaire, comprenant un corps (11) et un couvercle (12). Les géométries combinées du corps (11) et du couvercle (12) permettent leur emboîtement l'un dans l'autre. Lorsque le corps (11) et le couvercle (12) sont emboîtés, le volume intérieur du tube réactionnel (1) est isolé de l'atmosphère extérieure, la jonction étant étanche aux liquides et aux gaz. Le tube réactionnel (1) comprend un volume de liquide (13), dans lequel est au moins partiellement immergée une sonde thermique (14), la sonde thermique (14) étant prolongée par un moyen de liaison (15) à un moyen de réception du signal susceptible d'être généré par la sonde thermique (14), représenté sur la figure 1A.

Par « bloc thermique », on entend selon l'invention le moyen chauffant d'un thermocycleur PCR comprenant les puits dans lesquels sont placés les tubes contenant le mélange réactionnel pour réaliser les réactions PCR. En général, le bloc thermique consiste en un moyen chauffant par effet Peltier. Selon les modes de réalisation, les blocs thermiques peuvent comprendre 24, 30, 40, 48, 60, 96 ou 384 puits destinés à recevoir les tubes de mélange réactionnel pour PCR.

Par « enceinte thermiquement contrôlée», on entend selon l'invention une chambre à l'intérieur de laquelle demeure un carrousel comportant les puits dans lesquels il est prévu d'insérer les tubes ou capillaires contenant le mélange réactionnel pour réaliser une réaction de PCR. La régulation thermique de cette enceinte est assuré par de l'air pulsé. Selon les modes de réalisation, les enceintes thermiquement contrôlées sont équipées de carrousels qui peuvent comprendre 32, 36, 72 ou 99 puits destinés à recevoir les tubes ou capillaires de mélange réactionnel pour PCR.

Par « élément de régulation thermique », on entend selon l'invention l'un quelconque des éléments chauffants, en général par effet Peltier, contenus dans un bloc thermique. Le bloc thermique d'un thermocycleur pour PCR comprend dans la majorité des cas une pluralité d'éléments de régulation thermique. En général, chaque élément de régulation thermique est régulé de manière indépendante de tous les autres. Un bloc thermique comprend couramment de deux à huit éléments de régulation thermique. Une grande partie des thermocycleurs sont équipés d'un bloc thermique comprenant 2, 4, 6 ou 8 éléments de régulation thermique indépendants.

A l'étape b), on entend par « conditions normales d'utilisation », les conditions d'utilisation pour lesquelles ledit thermocycleur a été conçu par le constructeur. Plus précisément, les conditions normales d'utilisation englobent une utilisation du thermocycleur « capot fermé », c'est-à-dire dans les conditions de régulation et d'isolation thermique prévues par le constructeur, en évitant tout artéfact dû aux déperditions thermiques, qui sont observées avec les systèmes connus qui sont conçus pour être utilisés avec le thermocycleur en conditions « capot ouvert ».

Comme cela sera illustré en détail ultérieurement dans la description, certaines caractéristiques avantageuses des capteurs de mesure de l'invention permettent de tester tout type d'appareil thermocycleur, y compris les appareils thermocycleurs équipés de moyens automatisés de placement des tubes de mélange réactionnel au contact du bloc thermique et dans lesquels le bloc thermique est inaccessible à l'utilisateur.

A l'étape b), on met en fonctionnement le thermocycleur pour au moins un cycle de réaction PCR, à savoir pour un cycle normalement prévu pour réaliser successivement (i) une étape de dénaturation de l'ADN, (ii) une étape d'hybridation d'une ou plusieurs amorces nucléotidiques et (iii) une étape d'élongation des amorces préalablement hybridées. Classiquement, les valeurs de consigne de température pour les étapes (i), (ii) et (iii) sont respectivement de 95˚C, 60˚C et 72˚C. Une variété d'autres valeurs de consigne peuvent être choisies pour chacune des étapes (i), (ii) et (iii), du fait que l'objectif essentiel du procédé est de déterminer d'éventuelles différences entre les valeurs de consigne de température qui ont été initialement choisies pour programmer l'appareil thermocycleur et les valeurs réelles de température mesurées par les capteurs de mesure. Notamment les valeurs de consigne de température peuvent être fixées dans la programmation préalable du thermocycleur en fonction des températures que l'utilisateur envisage d'utiliser par la suite pour la réalisation effective d'un ensemble de cycles pour une réaction PCR.

Comme le comprend aisément l'homme du métier, le nombre de cycles de réaction PCR programmés ou réalisés, à l'étape b) du procédé n'est pas déterminant. Simplement, le nombre de données de mesure de température croît avec le nombre de cycles de réaction PCR réalisés à l'étape b). Dans les modes de réalisation particuliers du procédé dans lesquels on réalise une pluralité de cycles programmés pour une réaction PCR, on peut alors calculer, pour chaque instant caractéristique d'un cycle, et pour la totalité des cycles réalisés, une moyenne des valeurs de température mesurées par chacun des capteurs de mesure, ce qui est susceptible d'accroître la précision du test de régulation du bloc thermique.

Afin d'obtenir des résultats de test très fiables et reproductibles, on réalise à l'étape b) au moins trois cycles, mieux au moins cinq cycles programmés pour une réaction PCR. Pour l'obtention d'un résultat de test de très haute fiabilité et reproductibilité, on réalise par exemple au moins dix cycles programmés pour une réaction PCR.

A l'étape c), on entend, par « pluralité d'instants donnés », que des valeurs de mesure de température sont générées par chacun des capteurs de mesure à au moins trois instants importants de la durée d'exécution d'un cycle, respectivement (i) durant l'étape de dénaturation de l'ADN, (ii) durant l'étape d'hybridation des amorces nucléotidiques et (iii) durant l'étape d'élongation des amorces. Pour chacune des mesures précitées, l'instant de mesure est préférentiellement choisi au moment de l'étape dans lequel la valeur de température est normalement stabilisée, à savoir préférentiellement à la mi-durée de chacune de ces étapes.

En général, on mesure la température, à l'étape b) du procédé, à plus de trois instants donnés sur toute la durée des cycles programmés. Ces mesures sont effectuées aussi bien au cours des étapes précitées que pendant les phases de transition entre chacune des trois étapes précitées, afin de vérifier la régulation de chaque élément thermique du bloc thermique du thermocycleur également pendant les phases de variation programmée de la température, qui permet un test de la fiabilité du thermocycleur à la fois du point de vue du respect des valeurs de consigne de température et du respect des valeurs de consigne de point de départ et d'arrivée dans le temps des variations programmées de température, ainsi que du respect de la durée desdites variations programmées.

Dans la pratique, on réalise, à l'étape c) du procédé, et pour chacun des capteurs, au moins une centaine de mesures de la température pendant la durée d'un cycle de réaction PCR, de sorte que la « pluralité d'instants donnés » est en général au moins égal à cent. En fait, la cadence des mesures de la température pour les capteurs est usuellement en fréquence.

A l'étape c) du procédé, on réalise en général, avec chacun des capteurs une mesure des valeurs de température à une fréquence d'au moins 1 mesure par seconde, avantageusement de 2 mesures par seconde et préférentiellement de 4 mesures par seconde, et dans certains modes de réalisation du procédé au moins 10, 20 ou 40 mesures par seconde.

Préférentiellement, on dispose les capteurs de mesure, dans un même élément de régulation thermique, de telle manière qu'au moins un premier capteur de mesure soit positionné dans un puits localisé sur l'un des bords dudit élément de régulation thermique et qu'au moins un capteur de mesure soit positionné dans un puits localisé dans la partie centrale dudit élément de régulation thermique. Une telle disposition implique simultanément (i) le positionnement d'au moins deux sondes thermiques par élément de régulation thermique, (ii) le positionnement d'au moins une sonde thermique à chaque coin du bloc thermique et (iii) le positionnement d'au moins une sonde thermique au centre du bloc thermique.

La demanderesse a montré que, dans les dispositifs connus de mesure de la régulation des thermocycleurs PCR, la répartition géographique des sondes thermiques sur le bloc thermique n'était pas adaptée à la réalisation de mesures dont les résultats rendent fidèlement compte de l'éventuelle hétérogénéité de la justesse et la fidélité thermique.

La demanderesse a également montré que les prescriptions des normes en vigueur, telle que le norme ISO n° XP CEN ISO/TS 20836 2005 définissant les conditions d'essais de performance de thermocycleurs, et au moins en qui concerne les règles de localisation de sondes thermiques de test sur les blocs thermiques ne permettaient pas l'obtention de résultats reflétant fidèlement et/ou exhaustivement la justesse et la fidélité thermique réelle du bloc thermique testé. Cette norme introduit la notion « d'espace critique » mais ne définit pas de consignes précises de localisation des sondes thermiques sur le bloc thermique. Lorsque les espaces critiques ne sont pas connus, la norme prescrit de répartir les sondes thermiques de manière aléatoire dans les puits du bloc thermique à tester.

La demanderesse a aussi montré que, globalement, les puits localisés à la périphérie des blocs thermiques sont affectés d'une régulation thermique défectueuse et constituaient de ce fait un « espace critique ». On a en particulier observé selon l'invention qu'il existe un gradient de baisse des valeurs de température des mélanges réactionnels, du centre vers la périphérie des blocs thermiques. Les puits localisés dans chacun des coins du bloc thermique sont ceux pour lesquels il a été observé les défauts de régulation thermique les plus importants.

On a aussi montré selon l'invention que des hétérogénéités de régulation thermique existent également dans la partie centrale du bloc thermique, du fait que la majorité des blocs thermiques comprennent une pluralité d'éléments thermiquement régulés de manière indépendante, en général de 2 à 8 éléments de régulation thermique indépendants, pour chacun desquels existe aussi un « espace critique », en particulier pour les puits localisés à la périphérie desdits éléments de régulation thermique. En effet, les puits localisés à la périphérie d'un élément de régulation thermique donné d'un bloc thermique peuvent être en fait localisés dans la partie centrale dudit bloc thermique. On précise que, selon le modèle de thermocycleur considéré, la géométrie et le positionnement de chacun des éléments de régulation thermique constitutifs du bloc thermique diffèrent, de sorte qu'un schéma de disposition aléatoire de sondes thermiques sur un premier bloc thermique possédant un nombre donné d'éléments de régulation thermique a une faible probabilité de pouvoir être transposé avec succès pour tester un autre thermocycleur dont le bloc thermique comprend le même nombre d'éléments de régulation thermique.

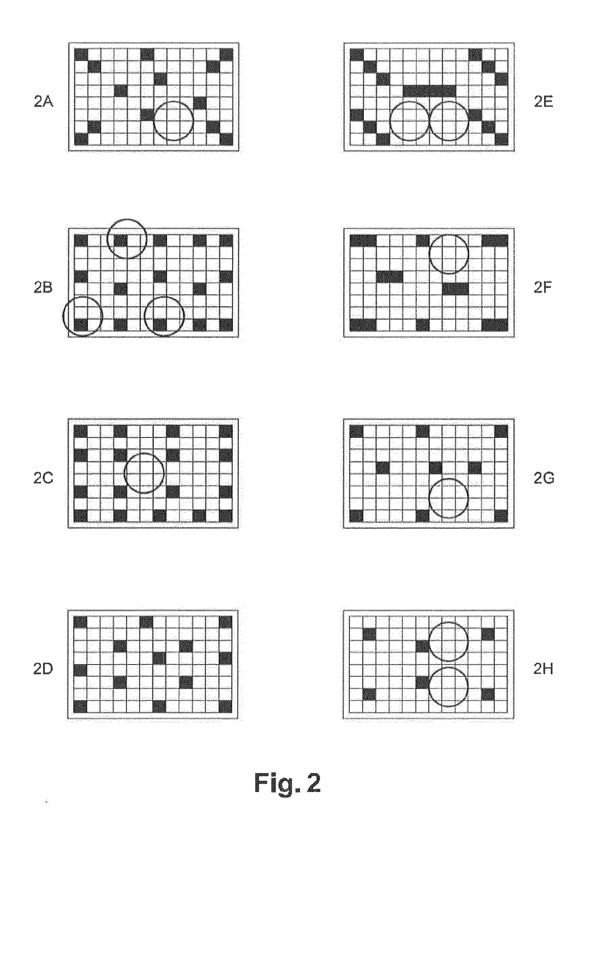

La demanderesse a montré que les prescriptions de positionnement des sondes sur le bloc thermique d'un thermocycleur à tester, y compris les prescriptions contenues dans la norme XP CEN ISO/TS 20836 2005 précitée, conduisaient à un positionnement des sondes inadapté à l'obtention de résultats reflétant fidèlement la régulation du bloc thermique testé. En utilisant les règles de positionnement de sondes prescrites par la norme ISO précitée, avec une variété de thermocycleurs équipés d'un bloc thermique de 96 puits, il s'est avéré que plusieurs des configurations prescrites pour la localisation des sondes thermiques ne permettaient pas de contrôler la totalité des éléments thermiques de régulation du bloc thermique, tout particulièrement pour les blocs thermiques comprenant 8 éléments de régulation thermique. La demanderesse a aussi montré que d'autres configurations de positionnement des sondes prescrites par la norme ne permettaient pas le contrôle d'un ou plusieurs des espaces critiques présents dans le bloc thermique. Les résultats d'essais réalisés par la demanderesse selon les règles contenues par la norme XP CEN ISO/TS 20836 2005 sont représentés sur la figure 2. La figure 2 représente des schémas de positionnement de sondes sur des blocs thermiques équipant une variété de modèles de thermocycleurs testés. Les carrés noirs illustrent la localisation des sondes thermiques. Les cercles illustrent, pour chaque thermocycleur testé, les zones de régulation thermique non contrôlées dans le test.

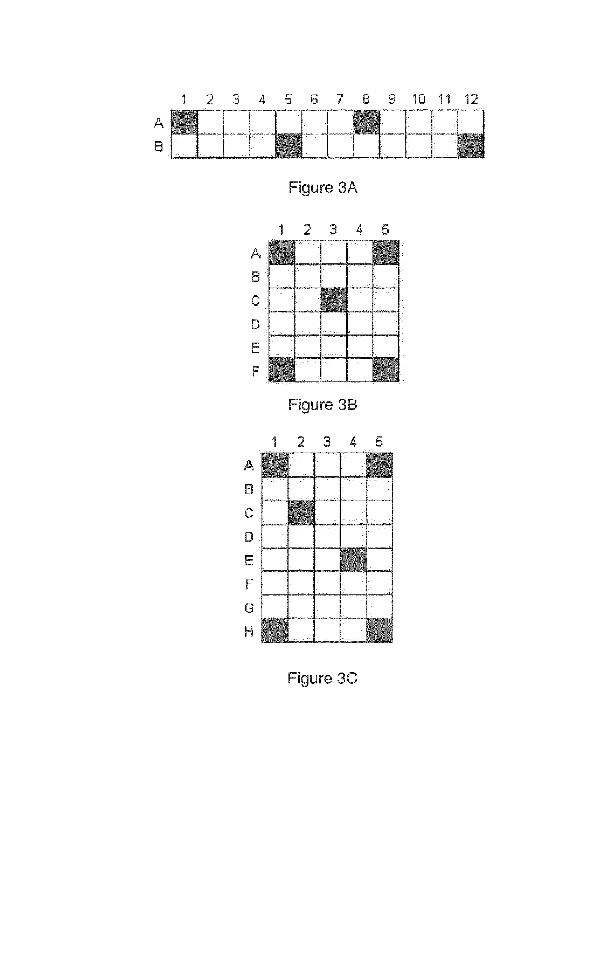

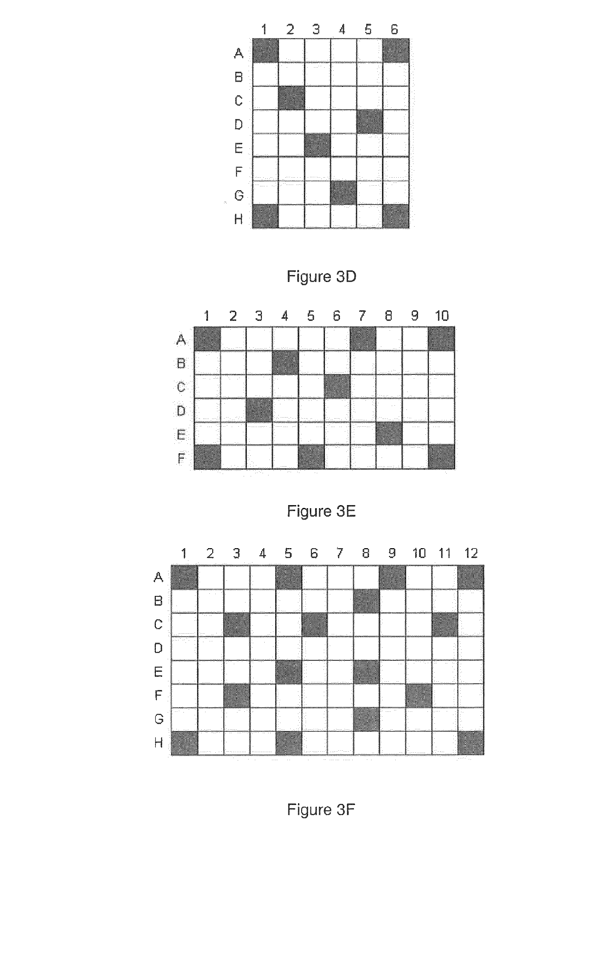

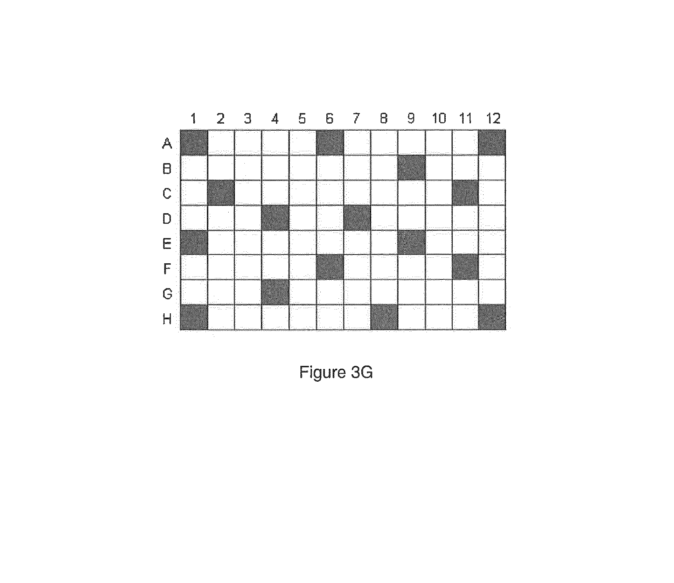

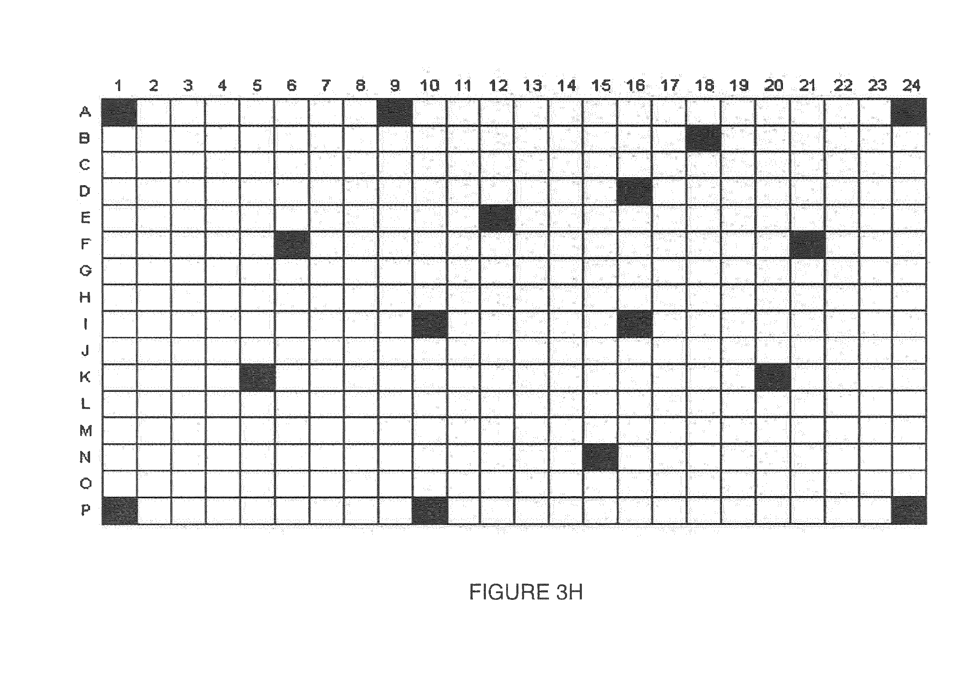

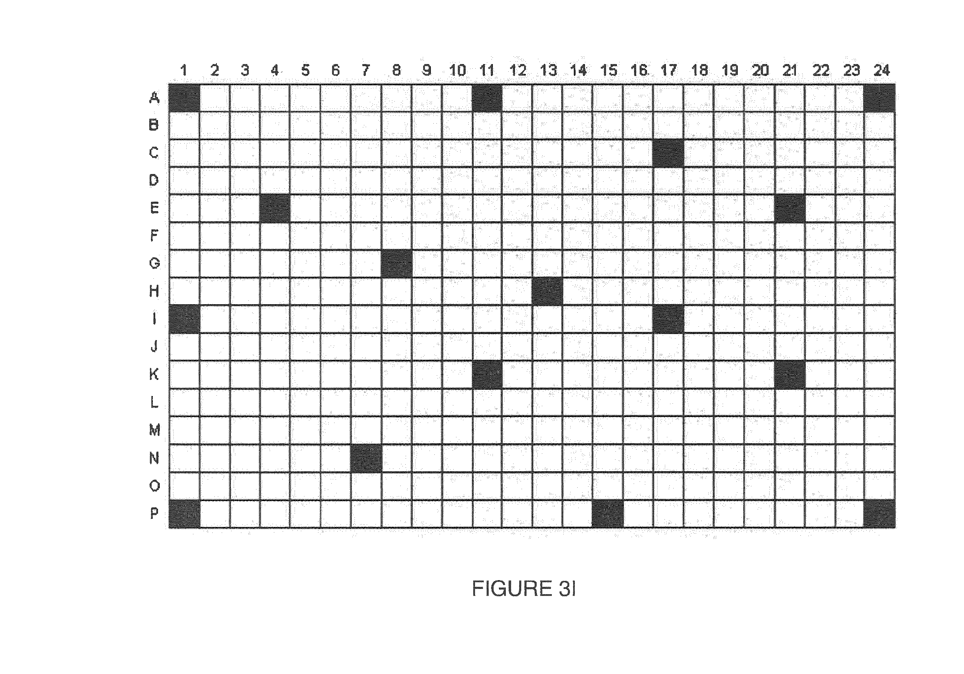

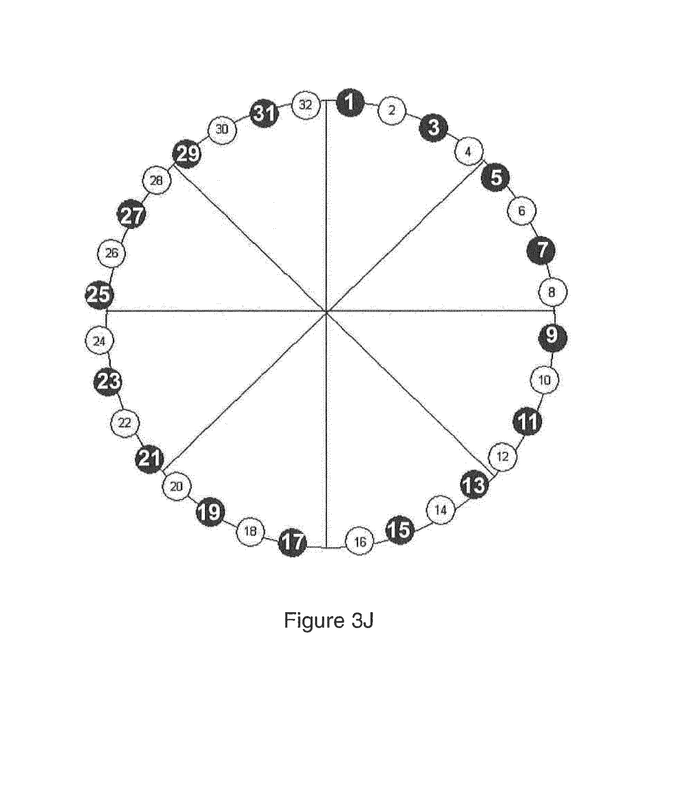

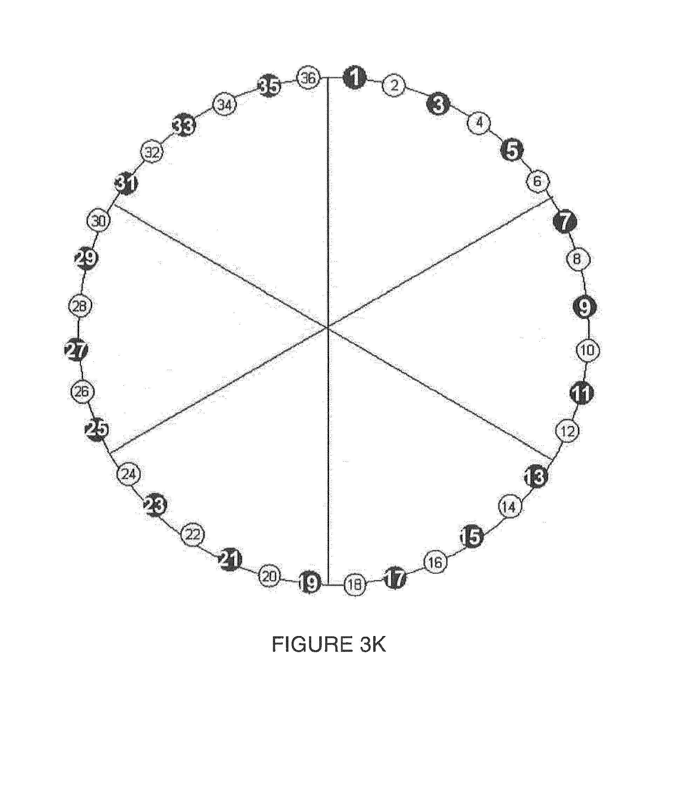

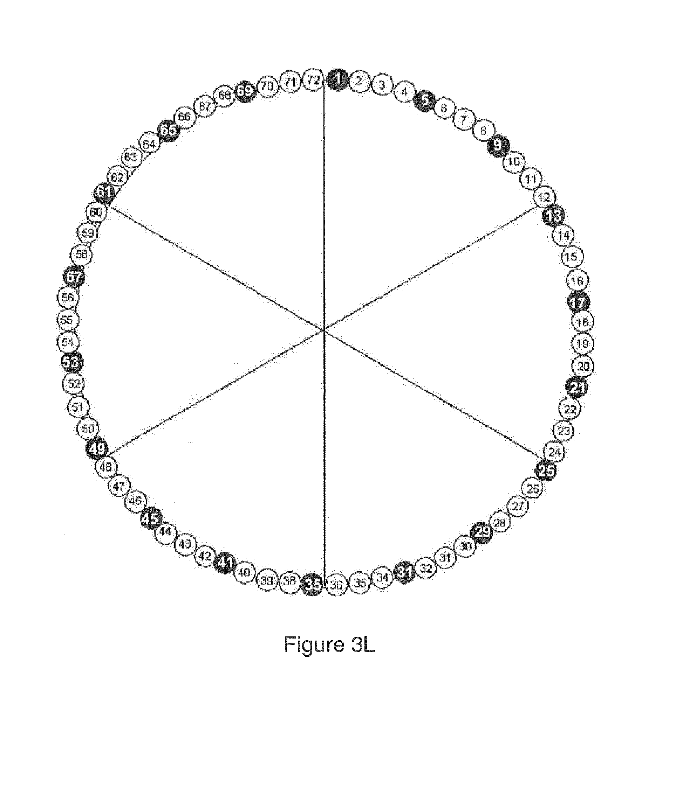

A partir des résultats obtenus selon les connaissances antérieures, la demanderesse a défini, après de longues recherches, des configurations de positionnement des capteurs de mesure de température permettant de tester de manière fiable, reproductible et exhaustive la justesse et la fidélité thermique du bloc thermique, quel que soit le type ou le modèle de thermocycleur testé. Les configurations préférentielles de positionnement des capteurs de mesure selon l'invention sont illustrées dans la figure 3. Dans les configurations illustrées dans la figure 3, sont positionnés au moins deux capteurs de mesure de température par élément de régulation thermique constitutif du bloc thermique testé, et au moins un capteur de mesure de température dans chacune des zones d'espace critique. La figure 3 (3A à 3I) illustre la configuration préférentielle de positionnement des capteurs de mesure pour des blocs thermiques comprenant 24, 30, 40, 48, 60, 96 ou 384 puits. Sur la base des règles précitées, et le cas échéant de l'illustration de la figure 3, l'homme du métier peut aisément déterminer une ou plusieurs configurations optimales de positionnement des capteurs de mesure de température pour tout type de bloc thermique, y compris pour des blocs thermiques de 24, 30, 40, 48, 60, 96 ou 384 puits.

Les configurations préférentielles de positionnement des capteurs de mesure de température, et notamment celles qui sont illustrées dans la figure 3 (3A à 3L), constituent un autre objet de la présente invention.

Dans les schémas des enceintes thermiquement contrôlées par air pulsé, les carrousels comportent de 32, 36, 72 ou 99 puits qui sont numérotés par convention de 1 à 32, de 1 à 36, de 1 à 72 ou de 1 à 99 de manière circulaire. La configuration préférentielle de positionnement des capteurs de mesure de température consiste donc en la configuration suivante, décrite par les coordonnées de positionnement des capteurs de mesure de température :

- 32 puits (Figure 3J) : 1, 3, 5, 7, 9, 11, 13, 15, 17, 19, 21, 23, 25, 27, 29, 31 (un puits sur deux)
- 36 puits (Figure 3K) : 1, 3, 5, 7, 9, 11, 13, 15, 17, 19, 21, 23, 25, 27, 29, 31, 33, 35 (un puits sur deux)
- 72 puits (Figure 3L) : 1, 5, 9, 13, 17, 21, 25, 29, 33, 37, 41, 45, 49, 53, 57, 61, 65, 69 (un puits sur 4)
- 99 puits : 1, 5, 10, 15, 20, 25, 30, 35, 40, 45, 50, 55, 60, 65, 70, 75, 80, 85, 90, 95 (un puits sur 5)

Dans les schémas des blocs de 24, 30, 40, 48, 60, 96 ou 384 puits (Figures 3A à 3I), chaque colonne comprend de 2 à 16 puits, qui sont numérotés par convention respectivement de A à P, dans les sens allant du haut vers le bas de la figure. De plus chaque rangée comprend de 5 à 24 puits, qui sont numérotés par convention respectivement de 1 à 24, dans les sens de la gauche vers la droite de la figure.

La configuration préférentielle de positionnement des capteurs de mesure de température selon l'invention sur un bloc thermique de 24, 30, 40, 48, 60, 96 ou 384 puits consiste donc en la configuration suivante, décrite par les coordonnées de positionnement des capteurs de mesure de température :

- 24 puits : 1 A, 5B, 8A, 12B
- 30 puits : 1 A, 1 F, 3C, 5A, 5F
- 40 puits : 1 A, 1 H, 2C, 4E, 5A, 5H
- 48 puits : 1 A, 1 H, 2C, 3E, 4G, 5D, 6A, 6H
- 60 puits : 1 A, 1 F, 3D, 4B, 5F, 6C, 7A, 8E, 10A, 10F
- 96 puits : 1 A, 1 H, 3C, 3F, 5A, 5E, 5H, 6C, 8B, 8E, 8G, 9A, 10F, 11C, 12A, 12H (stratégie 1), ou 1 A, 1 E, 1 H, 2C, 4D, 4G, 6A, 6F, 7D, 8H, 9B, 9E, 11C, 11 F, 12A, 12H (stratégie 2)
- 384 puits : 1 A, 1 P, 5K, 6F, 9A, 101, 10P, 12E, 15N, 16D, 161, 18B, 20K, 21 F, 24A, 24P (stratégie 1), ou 1 A, 1I, 1P, 4E, 7N, 8G, 11 A, 11 K, 13H, 15P, 17C, 171, 21 E, 21 K, 24A, 24P (stratégie 2).

Dans certains modes de réalisation du procédé, on dispose au moins trois, quatre ou même cinq capteurs de mesure par élément de régulation thermique constitutif du bloc thermique du thermocycleur testé.

Dans certains modes de réalisation avantageux, qui englobent les configurations préférentielles décrites ci-dessus, on dispose, à l'étape a) du procédé, un ensemble de 4, 5, 6, 8, 10 ou 16 capteurs de mesure dans les emplacements choisis du bloc thermique. Le demandeur a montré que la disposition d'un ensemble de 4, 5, 6, 8, 10 ou 16 capteurs de mesure dans des puits choisis du bloc thermique, (i) avec les conditions précitées de nombre minimum de capteurs par élément de régulation thermique, et facultativement (ii) avec les conditions de localisation des capteurs dans chacun desdits éléments de régulation thermique, constituait des dispositions universelles des capteurs de mesure de la température relative aux formats desdits blocs thermiques 24, 30, 40, 48, 60, 96 et 384 puits, utilisables quel que soit le thermocycleur testé présentant un nombre analogue de puits.

Ainsi, selon un autre aspect du procédé de l'invention, on dispose à l'étape a) respectivement pour lesdits blocs thermiques de 24, 30, 40, 48, 60, 96, 384 puits au moins 4, 5, 6, 8, 10, 16 et 16 capteurs de mesure de la température, et respectivement pour les carrousels de 32, 36, 72 et 99 puits desdites enceintes thermiquement contrôlées par air pulsé au moins 16, 18 et 18 capteurs de mesure de la température. D'autres dispositions avantageuses du procédé de l'invention sont décrites ci-après, en relation notamment avec les caractéristiques spécifiques de modes de réalisation particuliers des capteurs de mesure de la température utilisés.

Dans certains modes de réalisation, la sonde thermique du capteur de mesure consiste en une sonde à thermocouple d'un type connu. Ainsi, la sonde thermique est choisie notamment parmi les sondes à thermocouple de type E, J, K, N, T, R, S, B ou C et les sondes à résistance de type PT100, bien connues de l'homme du métier.

Préférentiellement, la sonde thermique est une sonde à thermocouple de type T constituée d'un alliage de cuivre et de nickel.

Selon d'autres caractéristiques avantageuses, le « liquide » contenu dans le tube réactionnel constitutif du capteur de mesure consiste en un liquide dont la composition mime celle d'un mélange réactionnel pour la réalisation d'une réaction PCR. Dans certains modes de réalisation, ledit liquide est de l'eau distillée ou déminéralisée. Dans d'autres modes de réalisation, ledit liquide est une solution tampon du type de celles qui sont couramment utilisées pour réaliser des réactions PCR. Dans encore d'autres modes de réalisation, ledit liquide consiste en une huile siliconée, ou mieux une solution tampon du type pour PCR dont l'interface supérieure est surmontée d'une couche d'huile siliconée.

On a en effet montré selon l'invention que la robustesse et la répétabilité du procédé était améliorée lorsque le liquide contenu dans le tube réactionnel constitutif du capteur de mesure de l'invention avait une composition ou une densité la plus proche possible de celle du mélange réactionnel utilisé pour la réalisation d'une réaction PCR.

Selon un autre aspect préférentiel, le « volume » de liquide contenu dans le tube réactionnel du capteur de mesure est d'au moins 10 $\mu$L. On a observé selon l'invention que l'utilisation de volumes de liquide inférieurs à 10 $\mu$L était défavorable à la fiabilité et à la reproductibilité du procédé.

Préférentiellement, toutefois, le volume de liquide contenu dans le tube réactionnel est similaire au volume de mélange réactionnel susceptible d'être ultérieurement utilisé pour réaliser effectivement des réactions PCR. Selon les cas, le volume de mélange réactionnel utilisé pour des réactions PCR varie de 10 $\mu$L à 50 $\mu$L.

En conséquence, le volume de liquide contenu dans le tube réactionnel d'un capteur de mesure selon l'invention contient préférentiellement de 10 $\mu$L à 50 $\mu$L dudit liquide.

Par « tube réactionnel », constitutif d'un capteur de mesure de température de l'invention, on entend tout tube d'un type connu qui est couramment utilisé pour réaliser des réactions PCR, qui comprend (i) un corps cylindrique, en général à bas tronconique, et (ii) un couvercle permettant d'isoler le volume intérieur du tube de l'atmosphère extérieure, et rendre ainsi le tube étanche aux liquides et aux gaz. Comme cela sera détaillé plus loin dans la description, les capteurs de mesure de température utilisés dans le procédé sont, du fait de leurs caractéristiques structurelles, adaptables à tout type de bloc thermique de thermocycleur, quelles que soient la forme et le volume des puits. Notamment, les capteurs de mesure utilisés dans le procédé de l'invention peuvent être utilisés avec des blocs thermiques munis de puits pour tubes de mélange réactionnel de très faible volume, y compris des tubes pour des volumes de 40 $\mu$L. Dans les modes de réalisation de l'invention dans lesquels le procédé est appliqué au réglage thermique d'un appareil thermocycleur PCR à enceinte thermiquement régulée par air pulsé, un « tube réactionnel » englobe un tube capillaire ou un tube plastique qui est conventionnellement utilisé dans ce type d'appareil.

De manière tout à fait préférée, le « moyen de liaison » de la sonde thermique au moyen de réception d'un signal généré par ladite sonde consiste en un câble électrique de diamètre réduit, permettant son passage soit entre le bord supérieur du tube réactionnel et le bord du couvercle en position fermée (Figures 1A, 1D), soit par un passage à travers le corps du tube (Figure 1 E), soit par un passage à travers la paroi du couvercle (Figure 1 C).

Un moyen de liaison du type câble électrique à diamètre réduit permet notamment son passage de l'intérieur vers l'extérieur du tube, sans gêner l'obturation dudit tube par le couvercle et donc sans affecter l'étanchéité de la jonction entre les bords du tube réactionnel et les bords du couvercle en position fermée.

Préférentiellement, on utilise un moyen de liaison consistant en un câble électrique ayant un diamètre d'au plus 2 millimètres de diamètre, et de manière tout à fait préférée d'un diamètre d'au plus 0,8 millimètres de diamètre et d'au minimum d'un diamètre de 0,2 millimètres.

Un autre avantage résultant de l'utilisation d'un moyen de liaison d'un diamètre réduit, pour un capteur de mesure de l'invention, est le très faible encombrement de la totalité des moyens de liaison constitutifs de l'ensemble de capteurs de mesure utilisés dans le procédé de test de régulation thermique. C'est le faible encombrement de la totalité des moyens de liaison des capteurs de mesure utilisés qui contribue à la possibilité de réaliser le procédé de l'invention « dans des conditions normales » d'utilisation de l'appareil thermocycleur. En effet, le faible encombrement des moyens de liaison rend possible l'exécution du test en conditions « capot fermé », quel que soit le type de thermocycleur testé, du fait que les moyens de liaison ne gênent pas la fermeture normale du thermocycleur. De même, le procédé de l'invention a été réalisé avec succès pour tester des thermocycleurs équipés de systèmes automatisés d'alimentation du bloc thermique avec les tubes réactionnels, pour lesquels le bloc thermique n'est pas accessible à l'utilisateur. Avec ce dernier type de thermocycleurs, la translation de l'ensemble des moyens de liaison vers l'intérieur de l'appareil, au moment de l'actionnement du mécanisme automatisé d'alimentation, ne gêne pas le déplacement des tubes réactionnels des capteurs de mesure de l'invention vers l'intérieur de l'appareil.

Selon encore un autre aspect avantageux résultant de l'utilisation, dans un capteur de mesure de l'invention, d'un moyen de liaison consistant en un câble électrique ayant diamètre réduit, est que la transmission du signal électrique généré par la sonde thermique, en général une sonde à thermocouple, s'effectue sans effet de charge. Par effet de charge, il est entendu que la masse globale des thermocouples ajoutés dans les tubes réactionnels est suffisamment faible pour ne pas influencer la régulation de température du bloc thermique.

Egalement, les dimensions réduites de l'ensemble sonde thermique/moyen de liaison sont adaptées à la fabrication de capteurs de mesure utilisables pour tester la justesse et la fidélité thermique de blocs thermiques équipés de puits pour tubes de très faibles volumes, y compris pour tubes de 40$\mu$L. Dans ces derniers modes de réalisation, on peut utiliser alors un tube de 40$\mu$L du commerce, comme tube réactionnel constitutif du capteur de mesure de température.

Dans un capteur de mesure de la température selon l'invention, la sonde thermique est maintenue en contact permanent avec le moyen de liaison, de préférence avec le câble électrique de diamètre réduit, au moyen d'une soudure appropriée, par exemple d'une soudure métallique, par exemple une soudure à l'or ou à l'argent.

Dans d'autres modes de réalisation d'un capteur selon l'invention, la sonde thermique (14) et le moyen de liaison (15) constituent deux parties distinctes d'un câble unique, par exemple d'un câble à thermocouple de type T en alliage Cuivre/Nickel.

Dans ces modes de réalisation, l'extrémité du câble immergée dans le mélange réactionnel possède la fonction de sonde thermique (14), la partie restante du câble, localisée entre l'extrémité immergée dans le mélange réactionnel et l'extrémité destinée à être connectée à un moyen de réception du signal a une fonction de moyen de liaison (15), puisque le câble est électroconducteur.

Préférentiellement, le câble électrique utilisé comme moyen de liaison résiste à une gamme de températures allant de 0˚C à 104˚C. Il en résulte que le capteur de mesure utilisé dans le procédé est adapté pour des tests de régulation thermique de thermocycleurs, quelles que soient les valeurs de consigne de température programmées pour la réalisation d'un cycle de réaction PCR, et en particulier quelle que soit la valeur de consigne de température programmée pour l'étape de dénaturation de l'ADN.

Selon un autre aspect, le câble électrique utilisé comme moyen de liaison possède une résistance mécanique telle qu'il n'est pas altéré par les contraintes mécaniques que subit la section dudit câble intercalée entre le bord supérieur du tube réactionnel et les bords du couvercle dudit tube réactionnel.

Les câbles électriques cités ci-dessus possèdent à la fois des caractéristiques de résistance thermique et de résistance mécanique requises. Afin d'optimiser les qualités de résistance mécaniques, dans certains modes de réalisation, lesdits câbles électriques comportent un gainage supplémentaire disposé au-dessus du gainage conventionnel initial des câbles. Les matières utilisées pour ce gainage supplémentaire sont celles qui présentent un bas coefficient de conductivité thermique et qui sont capables de résister à des températures supérieures à 105˚C. Les matières suivantes sont notamment, sinon exclusivement, concernées par l'application : polypropylène, PTFE, silicone.

Selon encore un autre aspect, le moyen de liaison a, par définition, une longueur suffisante pour assurer la transmission du signal électrique généré par la sonde thermique jusqu'au moyen de réception du signal. La longueur du moyen de liaison peut être aisément adaptée par l'homme du métier, selon le type de thermocycleur à tester et le type de disposition spatiale du moyen de réception du signal, par rapport audit thermocycleur. De manière générale, il est avantageux que la longueur du moyen de liaison soit adaptée pour tester tout type de thermocycleur, dans la plupart des dispositions spatiales du moyen de réception du signal, par rapport duit thermocycleur. Dans la pratique, une longueur du moyen de liaison d'au moins 50 centimètres peut être employée dans la plupart des situations de test. Une longueur d'au moins 50 centimètres englobe des longueurs d'au moins 60, 70, 80, 90, 100, 110, 120, 130, 140, 150, 160, 170, 180, 190 et 200 centimètres. On a montré selon l'invention que l'utilisation d'un moyen de liaison d'au moins 100 centimètres est adaptée pour la fabrication de capteurs de mesure selon l'invention qui peuvent être utilisés pour tester tout type de thermocycleur PCR, y compris les thermocycleurs équipés de moyens automatiques de placement des tubes de mélange réactionnel sur le bloc thermique.

La possibilité de faire passer le moyen de liaison à travers la paroi du bouchon (Figure 1 C), ou à la jonction entre les bords supérieurs du corps du tube réactionnel et les bords inférieurs du couvercle (Figure 1A, 1D), emboîtés l'un dans l'autre en position fermée, ou à travers la paroi du corps dudit tube réactionnel (Figure 1 E), rend possible l'utilisation de capteurs de mesure parfaitement adaptés au type de thermocycleur à tester, dont la fabrication est simple et qui sont d'un prix de revient réduit.

En effet, le passage d'un premier test d'un thermocycleur possédant un bloc thermique équipé d'un premier type de puits donné, à un second test d'un thermocycleur équipé d'un second type de puits donné distinct du précédent, peut ne nécessiter qu'une opération de retrait de l'ensemble sonde thermique/moyen de liaison d'un premier type de tube réactionnel adapté au premier type de puits, à un second type de tube réactionnel adapté au second type de puits donné, qui sera rempli du volume choisi de liquide.

En d'autres termes, le procédé pour tester la justesse et la fidélité thermique selon l'invention sur différents types d'appareils thermocycleurs peut être réalisé sans nécessiter un remplacement complet du matériel constitutif d'un premier capteur de mesure requis à un second capteur de mesure requis.

Ces avantages spécifiques sont procurés avec les capteurs de mesure de l'invention du fait que :

- la fabrication d'un capteur de mesure selon l'invention ne requiert aucune modification spécifique du tube réactionnel qui est utilisé, puisque l'ensemble sonde thermique/moyen de liaison est simplement introduit dans ledit tube, et
- la fabrication d'une série de capteurs de mesure pour des thermocycleurs d'un type distinct ne requiert pas obligatoirement l'utilisation d'autant d'ensembles sonde thermique/moyen de liaison que de capteurs de mesure désirés.

Pour la fabrication d'un capteur thermique selon l'invention, l'ensemble sonde thermique/moyen de liaison est introduit dans le tube réactionnel de telle manière que l'extrémité libre de la sonde thermique, qui est opposée à l'autre extrémité en contact avec le moyen de liaison, soit au moins partiellement immergée dans le liquide contenu dans ledit tube réactionnel.

Comme cela est illustré dans les exemples, la mise en oeuvre du procédé pour tester la régulation d'un bloc thermique ou d'une enceinte thermiquement contrôlée par air pulsé de thermocycleur peut être appliquée avec succès pour déterminer les conditions réelles de régulation thermique de l'appareil. La demanderesse a en effet observé qu'il existe des différences significatives, parfois très importantes, au moins concernant la justesse et la fidélité thermique, entre les caractéristiques de performance d'un thermocycleur décrites dans la notice technique du fabricant et les caractéristiques qui ont été mesurées en conditions réelles d'utilisation, par le procédé de l'invention. Ainsi, le procédé de l'invention permet désormais à l'utilisateur d'un thermocycleur de déterminer précisément la totalité des zones d'intérêt du bloc thermique testé. En particulier, grâce au procédé de l'invention, l'utilisateur est désormais informé, pour toutes les zones d'intérêt du bloc thermique testé, et pour la totalité de la durée du test comprenant un ou plusieurs cycles de réaction PCR, des écarts de température éventuels entre les valeurs de consigne initialement programmées et les valeurs réelles. En conséquence, grâce au procédé de l'invention, l'utilisateur a désormais accès non seulement aux valeurs de température réelles des étapes de dénaturation, d'hybridation et d'élongation, mais également aux valeurs de températures à une pluralité d'instants durant les phases programmées de variation de température, lorsque l'on passe successivement, dans une durée assez courte d'environ 30 secondes à 1 minute, (i) de l'étape de dénaturation à l'étape d'hybridation, (ii) de l'étape d'hybridation à l'étape d'élongation, puis (iii) de l'étape d'élongation à l'étape de dénaturation du cycle suivant de réaction PCR. Les conditions d'exécution des phases de variation de la température, qui sont communément désignées également phases de « ramping », influent de manière significative sur les résultats finals. Or, la demanderesse a montré qu'avec de nombreux thermocycleurs, les températures réelles des mélanges réactionnels pouvaient être supérieures ou inférieures aux valeurs de consigne initialement programmées, et même, pour une même phase de « ramping », (i) supérieure puis inférieure aux valeurs de consigne ou bien (ii) inférieure puis supérieure aux valeurs de consigne, selon l'instant de la phase de « ramping » considéré, selon l'étape du cycle de réaction PCR considéré.

De manière générale, le procédé pour tester la régulation d'un bloc thermique ou d'une enceinte thermiquement contrôlée par air pulséde thermocycleur pour PCR selon l'invention permet à l'utilisateur de connaître les performances réelles d'un appareil thermocycleur pour PCR, ce qui permet à cet utilisateur d'adapter la programmation de l'appareil, et en particulier les valeurs de consigne de température, et le cas échéant également les valeurs de durée des phases de « ramping », afin de réaliser ultérieurement les réactions PCR selon des conditions proches des conditions absolues choisies.

Ainsi, le procédé pour tester la justesse et la fidélité thermique d'un thermocycleur pour PCR peut comprendre une étape d) additionnelle de détermination de défauts dans la justesse et la fidélité thermique du bloc thermique testé, et plus généralement dudit thermocycleur.

Par « détermination de défauts dans la justesse et la fidélité thermique», on entend essentiellement un calcul de comparaison entre (i) des valeurs de consigne avec lesquelles le thermocycleur a été programmé et (ii) les valeurs réelles de fonctionnement mesurées à l'étape c) du procédé.

La comparaison entre les valeurs de consigne et les valeurs réelles mesurées permet le calcul des « performances » du thermocycleur testé.

Les « performances » d'un thermocycleur pour PCR englobent (i) l'exactitude de température, (ii) l'uniformité de la température et (iii) la vitesse de « ramping ».

L'exactitude de température consiste en la moyenne des valeurs de température mesurées par les différents capteurs de mesure, à un instant "t" donné. L'exactitude de température peut être calculée selon la formule (I) suivante :

$$E = Tprog - Tmoy \text{ (I)},$$

avec :

- E qui signifie la valeur d'exactitude de température, par exemple exprimée en degrés Celsius,
- *Tprog* qui signifie la valeur de consigne de température programmée sur le thermocycleur,
- *Tmoy* qui signifie la valeur moyenne de température mesurée par la totalité des capteurs de mesure audit instant « t », qui peut aussi être exprimée en degrés Celsius,

étant entendu que la valeur de température moyenne *Tmoy* est elle-même calculée selon la formule (II) suivante :

$$Tmoy = \sum_{i=1}^{n} \frac{Tsonde(i)}{n}, \text{ (II)}$$

avec

- *Tsonde*(i) qui signifie la valeur de mesure de température mesurée par le capteur de mesure (i), et
- n qui est un entier égal au nombre de capteurs de mesure de température disposés sur le bloc thermique ou dans l'enceinte thermiquement contrôlée par air pulsé.

L'uniformité de température consiste en l'écart entre la valeur de température maximale et la valeur de température minimale mesurée par les capteurs de mesure à un instant « t ». La valeur d'uniformité de température peut être calculée selon la formule (III) suivante :

$$U = Tmax - Tmin \text{ (III)},$$

avec

- U qui est la valeur d'uniformité de température, qui peut être exprimée en degrés Celsius,
- Tmax qui est la valeur de température maximale mesurée par les capteurs de mesure ausit instant « t », et
- Tmin qui est la valeur de température minimale mesurée par les capteurs de mesure audit instant « t ».

La vitesse de « ramping » consiste en le temps nécessaire pour passer d'un valeur de température programmée à une autre. La vitesse de « ramping » peut être calculée selon la formule (IV) suivante :

$$\text{Vitesse moyenne de ramping} = \left| \frac{T_1 - T_2}{t_1 - t_2} \right| \text{ (IV)},$$

avec :

- « Vitesse moyenne de ramping » qui est la valeur de vitesse de « ramping », qui peut être exprimée en degrés Celsius par seconde,
- $T_1$ qui est la valeur de température mesurée à l'instant $t_1$, et
- $T_2$ qui est la valeur mesurée à l'instant $t_2$

L'invention a aussi pour objet un capteur de mesure de la température d'un puits de bloc thermique ou de carrousel d'une enceinte thermiquement contrôlée par air pulsé d'un thermocycleur PCR comprenant :

(i) un tube réactionnel adapté au type de bloc thermique testé,
(ii) un volume choisi de liquide emplissant au moins partiellement ledit tube réactionnel
(iii) une sonde thermique au moins partiellement immergée dans ledit liquide, et
(iv) au moins un moyen de liaison de ladite sonde thermique à un moyen de réception d'un signal généré par ladite sonde thermique,

D'autres caractéristiques avantageuses du capteur de mesure de température selon l'invention ont été détaillées précédemment dans la présente description.

La présente invention a aussi pour objet un système pour tester la justesse et la fidélité thermique d'un appareil thermocycleur pour PCR, ledit système comprenant :

a) une pluralité de capteurs de mesure de la température tels que définis ci-avant, et
b) au moins un moyen de réception du signal de mesure de température généré par chacun desdits capteurs,

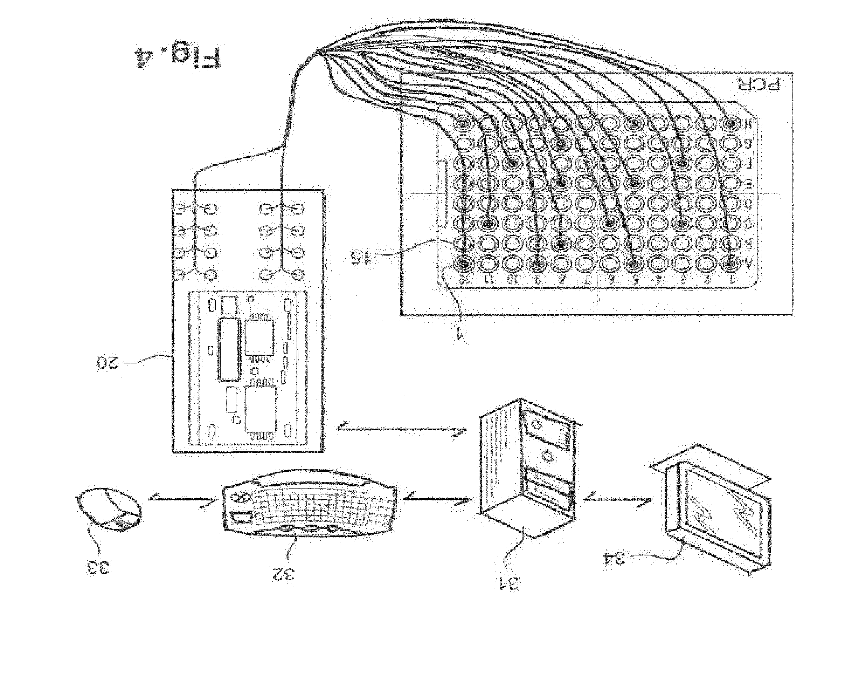

Le système pour tester la justesse et la fidélité thermique d'un bloc thermique de thermocycleur ou d'une enceinte thermiquement contrôlée par air pulsé pour PCR est décrit ci-après, en référence au mode de réalisation de ce système qui est illustré sur la figure 4.

Ledit système comprend une pluralité de capteurs de mesure (1), dont les moyens de liaison (15) sont reliés, à leur extrémité opposée à la sonde thermique dudit capteur (1), à un moyen de réception (20) du signal de mesure de température généré par ledit capteur (1).

On précise que, sur le schéma de la figure 4, les capteurs de mesure (1) sont disposés dans divers puits d'un bloc thermique (41) qui équipe le thermocycleur (40) testé. Sur la figure 4, bien que cela ne soit pas représenté de manière réaliste, on doit comprendre que, pour la mise en oeuvre du procédé de l'invention, le thermocycleur (41) est en conditions normales de marche, c'est à dire « capot fermé », ce qui est rendu possible par le diamètre réduit, préférentiellement inférieur à 1 millimètre, de chacun des moyens de liaison (15), et donc du très faible encombrement du faisceau constitué de la pluralité des moyens de liaison (15).

Le moyen de réception (20) du signal peut consister en un type quelconque de dispositif adapté à la réception d'un signal électrique généré par une sonde thermique, préférentiellement une sonde à thermocouple. Préférentiellement, on utilise un moyen de réception du signal comprenant un module de conversion du signal analogique délivré par chacune des sondes thermique en un signal numérique qui peut ensuite être traité dans un calculateur numérique approprié. Préférentiellement, on utilise un moyen de réception du signal adapté pour le traitement du signal généré par chaque capteur de mesure plusieurs fois par seconde. On utilise de préférence un moyen de réception apte à traiter le signal reçu de chaque capteur de mesure à une fréquence d'acquisition et de traitement d'au moins 5 points de mesure par seconde, ce qui inclus au moins 10, 15, 20, 25, 30, 35, 40, 45 ou au moins 50 points de mesure par seconde. Par définition, on choisit un moyen de réception apte à traiter le signal provenant de la totalité des capteurs de mesure de température utilisés dans le procédé. Par exemple, on peut utiliser un moyen d'acquisition du type NetDaq@ 2640A commercialisé par la société Fluke,

Le moyen de réception (20) du signal est relié à l'unité centrale (31) d'un calculateur numérique. Ledit calculateur numérique comprend, outre l'unité centrale (31), au moins un moyen d'entrée de données tels qu'un clavier (32) ou un dispositif de pointage (33), également reliés à l'unité centrale (31).

Le calculateur numérique comprend au moins un processeur de données et au moins un moyen de stockage de données.

Lorsqu'on met en oeuvre le procédé pour tester la justesse et la fidélité thermique d'un bloc thermique ou d'une enceinte thermiquement contrôlée par air pulsé de thermocycleur pour PCR, défini dans la présente description, les signaux transmis par les capteurs de mesure (1) vers le moyen de réception (20), s'ils sont de type analogiques, sont convertis en des signaux numériques. Les signaux numériques ainsi générés sont préférentiellement stockés dans la mémoire du calculateur numérique. Lesdits signaux numériques consistent en une collection ou en ensemble de valeurs de température mesurées par chacun des capteurs de mesure (1) à une pluralité d'instants « t ». L'ensemble de données peut donc se présenter sous forme de matrices à deux dimensions, dans lesquelles, à chaque valeur de température stockée, est associée la référence du capteur de mesure (1) qui a généré ladite valeur et l'instant « t » auquel ladite valeur a été générée.

Les données de mesure ci-dessus peuvent ensuite être traitées dans le calculateur numérique. Par exemple, la mémoire du calculateur numérique peur être chargée avec un programme d'ordinateur contenant un ensemble d'instructions logiques de calcul des performances du thermocycleur testé, par exemple respectivement les valeurs (i) d'exactitude de température, (ii) d'uniformité de température et (iii) de vitesse de « ramping ». On peut par exemple réaliser (i) un calcul de la performance d'un cycle de réaction PCR, évalué selon les valeurs d'exactitude et d'uniformité de température pour un cycle donné, (ii) un calcul de la performance sur une pluralité de cycles de réaction PCR, c'est à dire le cas échéant sur l'ensemble des cycles d'une réaction PCR complète, ce qui donne une valeur des performances calculées sur l'ensemble des cycles appliqués et sur le nombre de capteurs de mesure (1) utilisés, ou encore (iii) un calcul de la performance de zone, qui consiste en un calcul des valeurs d'exactitude de température et d'uniformité de température à partir des données de mesures de température générées par un capteur de mesure (1) donné, sur un cycle, une pluralité de cycles, ou la totalité des cycles inclus dans une réaction PCR. Ce dernier calcul permet d'évaluer de manière optimale l'éventuelle hétérogénéité de régulation thermique d'un bloc thermique ou d'une enceinte thermiquement contrôlée par air pulsé.

Un programme d'ordinateur comprenant un ensemble d'instructions pour le calcul d'un ou plusieurs paramètres de performance de la justesse et la fidélité thermique d'un bloc thermique de thermocycleur ou d'une enceinte thermiquement contrôlée par air pulsé pour PCR peut être réalisé par exemple par l'écriture de macro-commandes de traitement à partir de données de mesure stockées dans le fichier numérique d'un logiciel tableur courant.

Préférentiellement, on caractérise les performances du thermocycleur testé pour chaque palier de température.

Ainsi, grâce au procédé de l'invention, les utilisateurs sont désormais en mesure de contrôler de manière très précise les conditions de régulation thermique des thermocycleurs et de vérifier la stabilité desdites conditions de régulation thermique, au cours du temps.

Egalement, l'utilisateur, qui connaît désormais de manière précise les valeurs de dérive de température d'un thermocycleur donné, par rapport aux valeurs de consigne, est désormais en mesure de compenser les dérives de température mesurées avec le procédé et les capteurs de mesure de l'invention, pour réaliser la réaction PCR dans les conditions de température réelles identiques, ou quasi-identiques, à celles désirées.

Le procédé de test physique de la régulation d'un bloc thermique de thermocycleur ou d'une enceinte thermiquement contrôlée par air pulsé pour PCR peut être complété ou confirmé par un test de réglage biologique, bien connu de l'homme du métier.

L'invention concerne aussi un procédé pour tester la justesse et la fidélité thermique de thermocycleur pour PCR, comprenant les étapes suivantes :

a) réaliser un procédé de test de calibration physique tel que défini précédemment dans la présente description, et
b) réaliser un test de réglage biologique, à l'aide d'une pluralité de paires d'amorces ayant chacune une température optimale d'hybridation connue et distincte.

Pour les besoins de la présente invention, il a été mis au point spécifiquement un test de réglage biologique, destiné à être utilisé conjointement avec le test de réglage physique décrit précédemment, ou bien à être utilisé séparément du test de calibration physique.

Le test de réglage biologique de l'invention consiste, dans son principe, tout d'abord à déterminer une température seuil pour son utilisation sur un appareil thermocycleur pour PCR donné, puis à utiliser périodiquement ledit test, à la température seuil déterminée pour ledit thermocycleur, afin d'en vérifier la régulation du bloc thermique, sur l'ensemble du bloc thermique.

La température seuil à déterminer, dont les caractéristiques sont précisées plus loin, consiste en une température seuil d'hybridation d'amorces, dans une réaction PCR comprenant une pluralité de cycles de réaction PCR comprenant chacun successivement (i) une étape de dénaturation de l'ADN, (ii) une étape d'hybridation des amorces nucléiques, et (iii) une étape d'élongation des amorces.

Le test de réglage biologique de l'invention est basé sur la propriété d'une pluralité de paires d'amorces nucléiques à hybrider sur une cible ADN, chacune à une température théorique « Tm », ou température de fusion, spécifique, la succession de paires d'amorces hybridant à des températures de fusion distantes d'environ 5˚C.

Préférentiellement, dans le test de réglage de l'invention, on utilise trois ou quatre paires d'amorces dont les valeurs de température d'hybridation optimales sur l'ADN cible, dans la solution réactionnelle utilisée, sont respectivement de 50 ˚C, 55 ˚C, 60 ˚C et 65 ˚C.

La première étape du procédé de réglage de l'invention consiste à déterminer, spécifiquement pour l'appareil thermocycleur testé, une valeur de « température seuil », qui est la valeur de température d'hybridation mesurée par l'appareil à laquelle on détecte la génération, à partir de l'ADN cible, d'amplicons avec chacune des paires d'amorces utilisées.

Comme mélange réactionnel, on utilise un milieu comprenant :

- une solution tampon pour PCR,
- un ADN cible à amplifier,
- les trois ou quatre paires d'amorces ayant des températures optimales d'hybridation distinctes, et
- une ADN polymérase themorésistante, par exemple une Taq polymérase.

L'appareil thermocycleur à tester est préparé en programmant les températures désirées pour chacune des étapes respectives de dénaturation de l'ADN, d'hybridation des amorces nucléiques et d'élongation des amorces nucléiques.

Lorsque, comme dans les exemples 2 et 3, on utilise des amorces nucléiques contenant un marqueur fluorescent, la valeur de la « température seuil » est la valeur de température d'hybridation programmée dans le thermocycleur, pour laquelle on détecte simultanément de la fluorescence pour les amplicons générés par élongation de l'ADN à partir de chacune des paires d'amorces utilisées.

La visualisation de l'occurrence d'une réaction d'amplification avec chaque paire d'amorces peut être effectuée par (i) migration électrophorétique sur gel de la totalité de l'ADN amplifié, puis (ii) détection par fluorescence des bandes d'ADN correspondant aux amplicons générés par l'élongation de chacune des paires d'amorces nucléiques.

Préférentiellement, pour l'étape de détermination de la température seuil, on met en oeuvre le thermocycleur de manière à générer un gradient de température d'hybridation entre les puits successifs, afin de réaliser, avec le milieu réactionnel décrit ci-dessus, des réactions d'amplification simultanées avec une gamme de températures d'hybridation croissante pour des puits successifs distincts, ladite gamme de températures d'hybridation couvrant au moins la partie médiane de la gamme de températures d'hybridation optimale couverte par l'ensemble des paires d'amorces nucléiques utilisées, c'est à dire si possible dans une gamme de températures d'hybridation programmées dans le thermocycleur allant de 56°C à 60°C.

Préférentiellement, on programme l'appareil thermocycleur pour PCR pour réaliser un gradient de températures d'hybridation dont la borne inférieure est d'au moins 45°C, ce qui inclut au moins 46 °C, 47 °C, 48 °C, 49 °C, 50 °C, 51 °C, 52 °C, 53 °C, 54 °C, 55 °C et 56 °C.

Préférentiellement, on programme l'appareil thermocycleur pour PCR pour réaliser un gradient de températures d'hybridation dont la borne supérieure est d'au plus, 70°C, ce qui inclut au plus 65°C, 64°C, 63°C, 62°C et 61 °C.

Par exemple, le thermocycleur peut être programmé pour réaliser un gradient de températures d'hybridation allant de 56°C à 60°C.

Préférentiellement, la température de dénaturation programmée dans le thermocycleur est de 95 °C.

Préférentiellement, la température d'élongation programmée dans le thermocycleur est de 72 °C.

La valeur de « température seuil », pour l'appareil thermocycleur pour PCR testé, est la température d'hybridation mesurée par l'appareil thermocycleur des puits pour lesquels est observée l'amplification de l'ADN cible avec chacune des trois ou quatre paires d'amorces.

Préférentiellement, pour mettre en oeuvre le procédé de réglage biologique de thermorégulation d'un thermocycleur PCR, on utilise les paires d'amorces suivantes :

(i) une paire d'amorces de séquences SEQ ID N°1 et SEQ ID N°2, ayant une température optimale d'hybridation de 50°C,
(ii) une paire d'amorces de séquences SEQ ID N°3 et SEQ ID N°4, ayant une température optimale d'hybridation de 55°C,
(iii) une paire d'amorces de séquences SEQ ID N°5 et SEQ ID N°6, ayant une température optimale d'hybridation de 60°C,
(iv) une paire d'amorces de séquences SEQ ID N°7 et SEQ ID N°8, ayant une température optimale d'hybridation de 65°C,

La paire d'amorces de séquences SEQ ID N° 7 et SEQ ID N° 8 a été spécifiquement mise au point pour l'amplification d'un fragment de 483 paires de bases sur le même gène d'un ADN humain, en particulier d'un ADN de placenta humain, avec une très grande reproductibilité. La paire d'amorces de séquences SEQ ID N° 7 et SEQ ID N° 8 constitue un objet de l'invention, ainsi que des compositions les contenant, en particulier des compositions destinées à l'utilisation dans un test de réglage biologique de la justesse et la fidélité thermique d'un appareil thermocycleur pour PCR.

Préférentiellement, pour mettre en oeuvre le procédé de réglage biologique de thermorégulation d'un thermocycleur PCR, on utilise de l'ADN cible de placenta humain. A titre illustratif, on peut utiliser de l'ADN de placenta humain commercialisé par la société Sigma, sous la référence n ° D4642.

Puis, après détermination de la température seuil pour le thermocycleur PCR testé, un essai de réglage peut être réalisé lorsque désiré, par exemple régulièrement dans le temps ou lorsqu'un dysfonctionnement est suspecté, afin de vérifier la régulation thermique du bloc thermique ou de l'enceinte thermiquement contrôlée par air pulsé dudit appareil.

Préférentiellement, on vérifie la justesse et la fidélité thermique du bloc thermique ou de l'enceinte thermiquement contrôlée par air pulsé dudit thermocycleur en programmant celui-ci pour une température identique pour la totalité des puits et en disposant des tubes du mélange réactionnel ci-dessus dans des puits répartis sur la surface dudit bloc thermique de manière qu'au moins deux tubes de mélange réactionnel sont disposés dans chacun des éléments thermiques, compris dans ledit bloc thermique, ou pour les enceintes thermiquement contrôlée par air pulsé aux emplacements prévus pour les tubes ou capillaires de PCR de manière qu'au moins la moitié de ces emplacements soient occupés par des tubes de mélange réactionnel. En général, chacun des éléments thermiques consiste, dans la presque totalité des appareils thermocycleurs, en chacun des éléments de chauffe à effet Peltier compris dans le bloc thermique.

Il résulte de l'exposé qui précède que la présente invention a également pour objet un procédé de réglage biologique de la régulation du bloc thermique d'un appareil thermocycleur pour PCR, comprenant les étapes suivantes :

a) déterminer, pour ledit appareil thermocycleur, une température seuil de réglage, ladite température seuil de réglage étant la température d'hybridation mesurée par le thermocycleur à laquelle est amplifié un ADN provenant de placenta humain par chacune des paires d'amorces suivantes : (i) une paire d'amorces de séquences SEQ ID N˚1 et SEQ ID N˚2, (ii) une paire d'amorces de séquences SEQ ID N˚3 et SEQ ID N˚4, (iii) une paire d'amorces de séquences SEQ ID N˚5 et SEQ ID N˚6 et (iv) plus ou moins une paire d'amorces de séquences SEQ ID N˚7 et SEQ ID N˚8, en présence d'une ADN polymérase thermorésistante, et

b) tester la régulation du bloc thermique ou de l'enceinte thermiquement contrôlée par air pulsé dudit appareil thermocycleur, (i) en réalisant une réaction PCR, à une température d'hybridation programmée égale à la température seuil déterminée à l'étape a), puis (ii) en vérifiant que, à ladite température seuil programmée, on amplifie ledit ADN cible avec chacune des trois ou quatre paires d'amorces définies à l'étape a).

Préférentiellement, on effectue l'étape b) en disposant des tubes du mélange réactionnel ci-dessus dans des puits répartis sur la surface dudit bloc thermique de manière qu'au moins deux tubes de mélange réactionnel sont disposés dans chacun des éléments thermiques, c'est-à-dire en général chacun des éléments de chauffe par effet Peltier, compris dans ledit bloc thermique.

L'invention concerne aussi un procédé de réglage biologique d'un thermocycleur pour PCR, par un test de la régulation du bloc thermique ou de l'enceinte thermiquement contrôlée par air pulsé dudit appareil thermocycleur, comprenant les étapes suivantes :

a) réaliser une réaction PCR, à une température d'hybridation programmée égale à une température seuil de réglage dudit thermocycleur, ladite température seuil de réglage étant la température d'hybridation mesurée par le thermocycleur à laquelle est amplifié un ADN provenant de placenta humain par chacune des paires d'amorces suivantes : (i) une paire d'amorces de séquences SEQ ID N˚1 et SEQ ID N˚2, (ii) une paire d'amorces de séquences SEQ ID N˚3 et SEQ ID N˚4, (iii) une paire d'amorces de séquences SEQ ID N˚5 et SEQ ID N˚6 et (iv) plus ou moins une paire d'amorces de séquences SEQ ID N˚7 et SEQ ID N˚8, en présence d'une ADN polymérase thermorésistante, et

b) vérifier que, à ladite température seuil programmée, on amplifie ledit ADN cible avec chacune des quatre paires d'amorces définies à l'étape a).

La présente invention a aussi pour objet une composition d'amorces nucléiques pour la réalisation d'un test de réglage biologique d'un appareil thermocycleur pour PCR, comprenant de manière combinées ou séparées les paires d'amorces nucléiques suivantes :

(i) une paire d'amorces de séquences SEQ ID N˚1 et SEQ ID N˚2, ayant une température optimale d'hybridation de 50 ˚C,

(ii) une paire d'amorces de séquences SEQ ID N˚3 et SEQ ID N˚4, ayant une température optimale d'hybridation de 55˚C,

(iii) une paire d'amorces de séquences SEQ ID N˚5 et SEQ ID N˚6, ayant une température optimale d'hybridation de 60 ˚C, et

(iv) une paire d'amorces de séquences SEQ ID N˚7 et SEQ ID N˚8, ayant une température optimale d'hybridation de 65˚C.

Préférentiellement, dans la composition ci-dessus, chaque paire d'amorces est incluse dans un récipient séparé.

Préférentiellement, dans la composition ci-dessus, les amorces se présentent sous forme lyophilisée, la solution d'amorces étant utilisable, pour la réalisation d'un test de réglage biologique d'un appareil thermocycleur PCR, après reconstitution par ajout d'eau distillée déminéralisée, ou bien d'une solution tampon appropriée, selon les cas.

Un autre mode de réalisation d'un procédé de réglage biologique selon l'invention est illustré dans l'exemple 3. Dans cet autre mode de réalisation d'un test de réglage biologique de l'invention, on détermine sur le thermocycleur la température seuil (Ts) à partir de laquelle l'amplicon du promoteur inclus and le plasmide pBR322, c'est à dire l'acide nucléique amplifié grâce aux amorces appropriées (p.ex ; les amorces de séquences SEQ ID N˚11 et SEQ ID N˚ 12), ne donne aucun signal d'amplification sur gel d'agarose. Ce test est sensible à 0.5˚C près.

L'utilisateur devra donc réaliser des essais de gradient de température pour l'étape d'hybridation des amorces pour déterminer cette température seuil limite. Ensuite, il réalisera un test PCR à trois températures fixes d'hybridation des amorces, respectivement :

- température 1 : Ts - 1 ˚C
- température 2 : Ts - 0.5˚C
- température 3 : Ts

Préférentiellement, pour l'étape de détermination de la température seuil, on met en oeuvre le thermocycleur de manière à générer un gradient de température d'hybridation entre les puits successifs, afin de réaliser, avec le milieu réactionnel décrit ci-dessous, des réactions d'amplification simultanées avec une gamme de températures d'hybridation croissante pour des puits successifs distincts, ladite gamme de températures d'hybridation couvrant au moins la partie médiane de la gamme de températures d'hybridation optimale couverte par l'ensemble des deux paires d'amorces nucléiques utilisées, c'est à dire si possible dans une gamme de températures d'hybridation programmées dans le thermocycleur allant de 58˚C à 64˚C.

Préférentiellement, on programme l'appareil thermocycleur pour PCR pour réaliser un gradient de températures d'hybridation dont la borne inférieure est d'au moins 55˚C, ce qui inclut au moins 55˚C, 56˚C, 57˚C, 58˚C, 59˚C, 60˚C, 61 ˚C, 62˚C, 63˚C et 64˚C.

Préférentiellement, on programme l'appareil thermocycleur pour PCR pour réaliser un gradient de températures d'hybridation dont la borne supérieure est d'au plus, 70˚C, ce qui inclut au plus 65˚C, 64˚C, 63˚C, 62˚C et 61 ˚C.

Par exemple, le thermocycleur peut être programmé pour réaliser un gradient de températures d'hybridation allant de 58˚C à 64˚C.

Préférentiellement, la température de dénaturation programmée dans le thermocycleur est de 95 ˚C.

Préférentiellement, la température d'élongation programmée dans le thermocycleur est de 72 ˚C.

La valeur de « température seuil », pour l'appareil thermocycleur pour PCR testé, est la température d'hybridation mesurée par l'appareil thermocycleur des puits pour lesquels est observée l'absence d'amplification de l'ADN cible pBR322 avec la paire d'amorces pBR322F01 et pBR322R03.

Préférentiellement, pour mettre en oeuvre le procédé de réglage biologique de thermorégulation d'un thermocycleur PCR, on utilise les paires d'amorces suivantes :

(v) une paire d'amorces de séquences SEQ ID N˚9 et SEQ ID N˚10, ayant une température optimale d'hybridation de 60˚C,

(vi) une paire d'amorces de séquences SEQ ID N˚11 et SEQ ID N˚12, ayant une température limite d'hybridation inférieure de 0.5˚C à la température seuil (Ts) à partir de laquelle l'amplicon du promoteur pBR322 ne donne aucun signal d'amplification sur gel d'agarose,

Ainsi, la présente invention concerne aussi un procédé de réglage biologique de la justesse et la fidélité thermique du bloc thermique ou d'une enceinte thermiquement contrôlée d'un appareil thermocycleur pour PCR, comprenant les étapes suivantes :

a) déterminer, pour ledit appareil thermocycleur, une température seuil (Ts) de réglage, ladite température seuil (Ts) de réglage étant la valeur de température commune où sont vérifiées les conditions (i) et (ii) ci-après :

(i) la température d'hybridation mesurée par le thermocycleur, à laquelle est amplifié un ADN plasmidique circulaire (« ADN cible ») par la paire d'amorces de séquences SEQ ID N˚9 et SEQ ID N˚10, en présence d'une ADN polymérase thermorésistante, et

(ii) la température limite d'hybridation mesurée par le thermocycleur, à laquelle n'est pas amplifié un ADN plasmidique circulaire (« ADN cible ») par la paire d'amorces de séquences SEQ ID N˚11 et SEQ ID N˚12, en présence d'une ADN polymérase thermorésistante, et

b) tester la justesse et la fidélité thermique du bloc thermique ou de l'enceinte thermiquement contrôlée dudit appareil thermocycleur, en réalisant les étapes suivantes :

b1) on réalise trois réactions PCR, à des températures d'hybridation programmée respectivement égales à (1) la température seuil (Ts) déterminée à l'étape a), (2) une température inférieure de 0,5˚C (Ts-0.5˚C) à la température seuil (Ts) déterminée à l'étape a), et (3) une température inférieure de 1 ˚C (Ts-1 ˚C) à la température seuil déterminée à l'étape a),

b2) on vérifie que, (1) auxdites températures (Ts-0.5˚C) et (Ts-1 ˚C), on amplifie ledit ADN cible avec chacune des deux paires d'amorces SEQ ID N˚9-10 et SEQ ID N˚11-12 et (2) à ladite température seuil (Ts) déterminée à l'étape a), on amplifie ledit ADN cible avec uniquement la paire d'amorces de séquences SEQ ID N˚9 et SEQ ID N˚10.

La présente invention est aussi relative à une composition d'amorces nucléiques pour la réalisation d'un test de réglage biologique d'un appareil thermocycleur pour PCR, comprenant deux paires d'amorces, respectivement la paire d'amorces de séquences SEQ ID N˚ 9-10 et la paire d'amorces de séquences SEQ ID N˚11-12.

La présente invention est en outre illustrée, sans y être limitée, par l'exemple suivant.

## EXEMPLES

## Exemple 1 : Détermination des profils des cinétiques de température

Différents tests de régulation thermique ont été réalisés, avec le procédé de l'invention, sur plusieurs thermocycleurs du commerce, afin de mieux évaluer les profils caractéristiques des cinétiques de réaction PCR.

Quels que soient les appareils testés, ce sont généralement les mêmes profils qui sont obtenus. Lorsque le mode de régulation du thermobloc ou de l'enceinte thermiquement contrôlée par air pulsé est correct, les profils de température sont linéaires et parfaitement proportionnés. Le début et la fin des plateaux sont clairement identifiables sur les profils cinétiques des figures 5A1 (étape de dénaturation), 5A2 (étape d'hybridation) et 5A3 (étape d'élongation).

Lorsque la justesse et la fidélité thermique du thermobloc ou de l'enceinte thermiquement contrôlée par air pulsé est hétérogène ou imprécise, une étape transitoire est apparente au début des plateaux de température. Sa durée est plus ou moins longue. Cette étape peut se caractériser sous la forme de pics positifs ou négatifs, par rapport à la température moyenne du plateau, comme cela est illustré sur les profils cinétiques des figures 5B1 (étape de dénaturation), 5B2 (étape d'hybridation) et 5B3 (étape d'élongation).

L'étape transitoire peut également se manifester sous la forme d'une montée ou d'une descente trop progressive de la température, de sorte qu'il est impossible de fixer un réel point de départ du plateau, comme cela est illustré sur les profils cinétiques des figures 5C1 (étape de dénaturation), 5C2 (étape d'hybridation) et 5C3 (étape d'élongation).

Le procédé, les capteurs de mesure, et plus généralement le système de mesure de la régulation d'un bloc thermique ou de l'enceinte thermiquement contrôlée par air pulsé d'un thermocycleur sont donc opérationnels.

Il a aussi été mis au point un programme de traitement numérique des données collectées par le moyen de réception des signaux provenant des capteurs de mesure.

## Exemple 2 : Description d'un test de calibration biologique [Stratégie 2]

Le test de calibration biologique décrit ci-après a été réalisé sur la base des informations contenues dans l'article de Yang et al (Use of multiplex polymerase chain reactions to indicate the accuracy of the annealing temperature of thermal cycling ; Analytical Biochemistry, Vol. 338 (2005) : 192-200)

Le test cible deux gènes différents du même tissu. L'ADN cible est un ADN de placenta humain commercialisé par la société SIGMA (*SIGMA*-Aldrich, St. Louis, MO ; référence D4642).

L'article de Yang et al (2005) décrivait une réaction PCR multiplex utilisant quatre couples d'amorces. Toutefois, la demanderesse a observé que la quatrième paire d'amorces décrites par Yang et al. (2005), désignée « 482 », n'était pas fonctionnelle

Le test décrit dans le présent exemple met en oeuvre trois des quatre paires d'amorces décrites par Yang et al. (2005), respectivement les paires d'amorces désignées « 200 », « 300 » et « 400 ».

La quatrième paire d'amorces, désignée « 480 », a été mise au point spécifiquement pour l'exemple. Plus précisément, la paire d'amorces désignée «480 » comprend une amorce spécifique nouvelle, l'amorce « ADN AS483 » de séquence SEQ ID N˚8, qui permet, lorsqu'elle est utilisée conjointement avec l'amorce « ADN S480 » de séquence SEQ ID N˚ 7, de générer un amplicon de 483 paires de bases.

200 : fragment à température d'hybridation optimale: 50˚C

Séquence ADN S200 : 5' CACACTTCATATTTACCCAT 3' (SEQ ID N ˚1)

Séquence ADN AS200 : 5' TTGTTTAATAGAGACGAAGG 3' (SEQ ID N˚2)

300 : fragment à température d'hybridation optimale: 55˚C

Sequence ADN S300 : 5' ATGGACATTTACGGTAGTGG 3' (SEQ ID N˚3)

Séquence ADN AS300 : 5' AAGTATTTCAATGCCGGTAG 3' (SEQ ID N˚4)

400 : fragment à température d'hybridation optimale: 60 ˚C

Séquence ADN S400 : 5' GCTAGCTGTAACTGGAGCCG 3' (SEQ ID N˚5)

Séquence ADN AS 400 : 5' GTCTGCTGAAACTGCCAACA 3' (SEQ ID N˚6)

480 : fragment à température d'hybridation optimale: 65˚C

Séquence ADN S480 : 5' GGCCTGCTGAAAATGACTGA 3' (SEQ ID N˚7)

Séquence ADN AS483 : 5' CAAAACAAAACAATATATACATTCCAA 3' (SEQ ID N˚8)

L'amorce AS483 est donc, dans le test spécifique de l'exemple, l'amorce complémentaire de S480 pour l'amplification d'un fragment de 483 pb sur le même gène au lieu de 480pb comme initialement prévu dans l'article de Yang et al. (2005).

On ajoute 5 à 30 ng d'ADN pur de placenta par puits.

**Tests en PCR point final**

Pour amplifier plusieurs ADN cibles dans les conditions limites d'amorçage et déceler les montées ou baisses de températures, une PCR multiplex est réalisée en Gradient de température d'hybridation. Le Gradient consiste à obtenir une température d'hybridation des amorces différentes soit sur chaque colonne, soit sur chaque ligne du bloc PCR (selon les modèles d'appareils PCR sur le marché).

Tableau 1 : composition du mélange réactionnel pour 3 ou 4 bandes

| Mélange pour 4 bandes : | | | Mélange pour 3 bandes : | | |
|---|---|---|---|---|---|
| | Cf | 1 réaction PCR (µL) | | Cf | 1 réaction PCR (µL) |
| Tampon (10X) | 1X | 2,5 | Tampon (10X) | 1X | 2 |
| $H_2Oup$ | | 4,83 | $H_2Oup$ | - | 9,18 |
| 200S (100µM) | 4µM | 1 | 200S (104,36µM) | 0,41µM | 0,08 |
| 200AS (100µM) | 4µM | 1 | 200AS (104,36µM) | 0,41µM | 0,08 |
| 300S (25µM) | 0,2µM | 0,2 | 300S (8,46µM) | 0,22µM | 0,52 |
| 300AS (25µM) | 0,2µM | 0,2 | 300AS (8,46µM) | 0,22µM | 0,52 |
| 400S (25µM) | 0,6µM | 0,6 | 400S (8,58µM) | 0,07µM | 0,16 |
| 400AS (25µM) | 0,6µM | 0,6 | 400AS (8,58µM) | 0,07µM | 0,16 |
| 480S (100µM) | 5µM | 1,25 | | | |
| 483AS (100µM) | 5µM | 1,25 | Solution Q**(5X) | 0,25X | 1 |
| $MgCl_2$ (50mM) | 2mM | 1 | $MgCl_2$ (50mM) | 2mM | 0,8 |
| dNTPs (25mM) | 0,375 mM | 0,375 | dNTPs (25mM) | 0,375 mM | 0,3 |
| *Taq* I (5U/µL) | 1 U/réaction | 0,2 | *Taq* 1 (5U/µL) | 1 U/réaction | 0,2 |
| TOTAL | | 15 | TOTAL | | 15 |
| Matrice ADN | à ajuster* | 5 | Matrice ADN | à ajuster* | 5 |
| Volume final | | 20 | Volume final | | 20 |

Cf : Concentration finale; S : Sens; AS ; antisens
* La quantité de cible matrice (ADN de placenta) doit être ajustée suivant la sensibilité du thermocycleur utilisé.
** la solution Q (commercialisée par la société Qiagen dans le kit de référence 203203) a été utilisée comme adjuvant pour défavoriser la fixation des amorces. Elle est composée principalement de bétaïne.

La préparation des solutions d'amorces est une étape essentielle pour la reproductibilité de l'expérience dans le temps. On s'attache à utiliser des pipettes adaptées aux volumes à pipeter, justes et reproductibles ayant subi un contrôle métrologique récent. On prend soin également de préparer un lot unique de chaque amorce (redosé de façon précise à l'aide d'un NanoVue™ ou NanoDrop™) à partir duquel on prélève des fractions aliquotes. Chaque fraction aliquote issue du lot de départ est utilisée pour un usage unique. Les concentrations finales des amorces ont été établies après avoir étudié et comparé les différentes concentrations en $MgCl_2$, amorces ainsi que différentes *Taq* polymerases.

Le programme PCR utilisé est décrit dans le tableau 2 le suivant :

Tableau 2 : paramètres de la réaction PCR

| | 95˚C | 5' | **x 1** |
|---|---|---|---|

(suite)

| | | | |
|---|---|---|---|
| | 95°C | 30" | **x 30** |
| **gradient** | **57 à 63°C** | 30" | |
| | 72°C | 30" | |
| | 72°C | 5' | **x 1** |

Les thermocycleurs de 1ere génération (avant 2006) ont une température de "ramping" max de 3°C/sec, alors que ceux de 2eme génération (après 2006) ont une température de ramping max de 4 °C/sec.

Toutefois, pour les appareils ne comportant pas d'option de gradient, la PCR sera réalisée une première fois aux deux températures théoriques encadrant la température seuil, puis à la température seuil de contrôle où les 3 ou 4 bandes sont présentes.

Tableau 3 : Exemple de schéma de bloc pour gradient:

| | 1 | 2 | 3 | 4 | 5 | 6 | 7 | 8 | 9 | 10 | 11 | 12 | |
|---|---|---|---|---|---|---|---|---|---|---|---|---|---|
| | | | | | | 3.1 Thermocycleur A | | | | | | | |
| A | | Plac D10 | | | | Plac D10 | | | | Plac D10 | | | **60°C** |
| B | | Plac D10 | NTC1 | | | Plac D10 | | | | Plac D10 | | | **59,8** |
| C | | Plac D10 | | | | Plac D10 | | | | Plac D10 | | | **59,5** |
| D | | Plac D10 | | | | Plac D10 | | | | Plac D10 | | | **58,9** |
| E | | Plac D10 | | | | Plac D10 | | | | Plac D10 | | | **58,2** |
| F | | Plac D10 | | | | Plac D10 | | | | Plac D10 | | | **57,6** |
| G | | Plac D10 | | | | Plac D10 | | | | Plac D10 | | | **57,2** |
| H | | Plac D10 | NTC 2 | | | Plac D10 | | | | Plac D10 | | | **57** |

| | 1 | 2 | 3 | 4 | 5 | 6 | 7 | 8 | 9 | 10 | 11 | 12 | |
|---|---|---|---|---|---|---|---|---|---|---|---|---|---|
| | | | | | | 3.2. Thermocycleur B | | | | | | | |
| A | | Plac D10 | Plac D10 | Plac D10 | Plac D10 | Plac D10 | Plac D10 | Plac D10 | Plac D10 | | | | |
| B | | NTC 1 | | | | | | | NTC 2 | | | | |
| C | | | | | | | | | | | | | |
| D | | Plac D10 | Plac D10 | Plac D10 | Plac D10 | Plac D10 | Plac D10 | Plac D10 | Plac D10 | | | | |
| E | | | | | | | | | | | | | |
| F | | | | | | | | | | | | | |
| G | | Plac D10 | Plac D10 | Plac D10 | Plac D10 | Plac D10 | Plac D10 | Plac D10 | Plac D10 | | | | |
| H | | | | | | | | | | | | | |

(suite)

| 3.2. Thermocycleur B | | | | | | | | | | | | |
|---|---|---|---|---|---|---|---|---|---|---|---|---|
| **1** | **2** | **3** | **4** | **5** | **6** | **7** | **8** | **9** | **10** | **11** | **12** | |
| 57°C | 57,1 | 57,5 | 58 | 58,7 | 59,4 | 60,2 | 60,9 | 61,5 | | | | |

NTC : Témoin négatif sans ADN (pour « No Template Control »).
Plac D10 : 30ng d'ADN de Placenta dilué 10 fois.

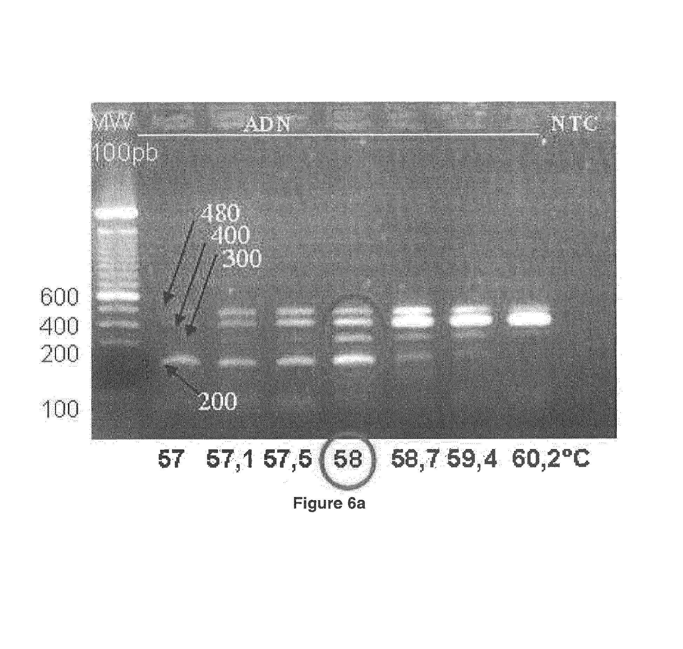

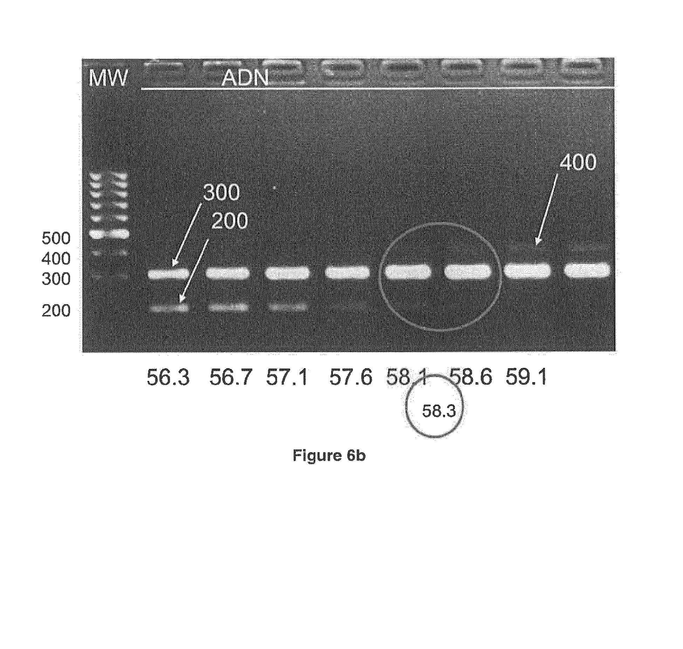

Les résultats sont représentés sur les figures 6 (a, b) (thermocycleur A) et 7 (a, b) (thermocycleur B), qui consistent en des clichés de migration des amplicons sur gel d'agarose à 2%. La migration par électrophorèse est réalisée à 120 Volts.

Sur la figure 6a, le point limite où les 4 bandes sont présentes à même intensité est à 58°C. On obtient 0,7°C avant seulement 3 bandes nettes (480, 400 et 200pb) et 0,7°C après seulement 2 bandes nettes (480 et 400 pb).

Ce test est sensible à une augmentation de température et à une diminution de température, à 0,7°C près.

Sur la figure 6b, le point limite où les 3 bandes sont présentes à même intensité est à 58,3°C à cheval entre 58,1 et 58,6°C. On obtient 0,7°C avant seulement 2 bandes nettes (400 et 200pb) et 0,7°C après seulement 2 bandes nettes (400 pb).

Ce test est sensible à une augmentation de température et à une diminution de température, à 0,7°C près.

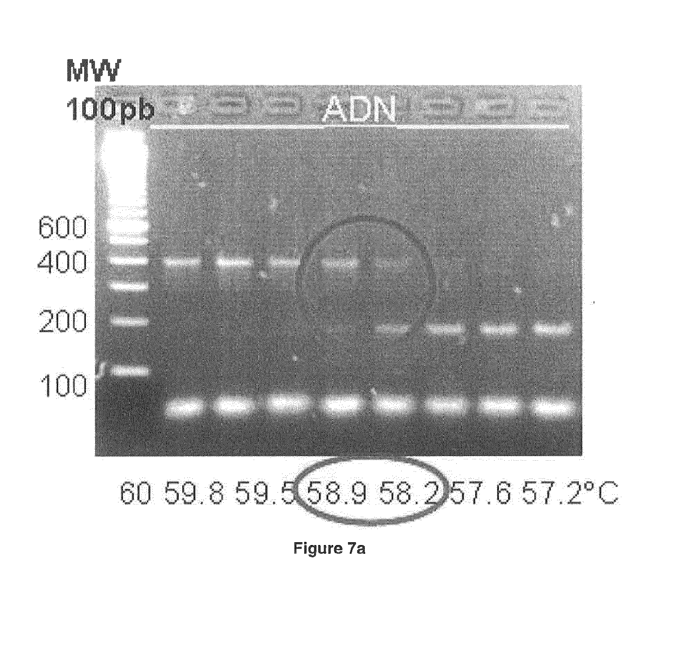

Sur la figure 7a, le point limite où les 4 bandes sont présentes à même intensité est entre 58,9 et 58,2°C. On obtient 0,7°C avant seulement 1 bande nette (400pb) et 0,7°C après seulement 1 bande nette (200pb). La bande à 300 pb est très faible et celle à 480 pb n'apparaît pas.

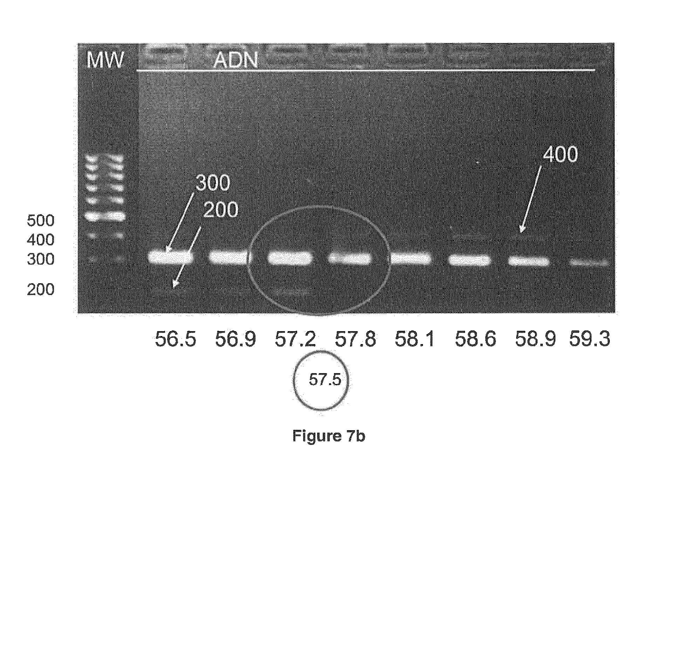

Sur la figure 7b, le point limite où les 3 bandes sont présentes à même intensité est entre 57,2 et 57,8°C. On obtient 0,6°C avant 2 bandes nettes (200 et 300pb) et 0,6°C après 2 bandes nettes (300 et 400pb). On a bien les 3 bandes attendues.

Ce test est différent d'un appareil à l'autre et d'un fabriquant à l'autre. En se basant sur le mélange réactionnel décrit plus haut, l'utilisateur doit établir une carte de contrôle pour chaque appareil à contrôler et déterminer ainsi la température seuil de l'appareil à laquelle on observe au moins 2 des 4 bandes.

La mise au point de cette carte de contrôle se réalise par ajustement de la vitesse de ramping des appareils dans un premier temps, puis de la concentration en matrice ADN si nécessaire, étant donné que le mélange réactionnel est optimisé pour avoir un bon équilibre entre les différentes cibles.

Une fois cette carte établie, et cette température seuil déterminée, l'utilisateur peut se limiter à réaliser une réaction PCR sur toute la plaque à cette température seuil, par exemple trimestriellement, ou bien lorsqu'il observe un résultat anormal.

Le gradient n'est nécessaire que pour la mise au point du test.

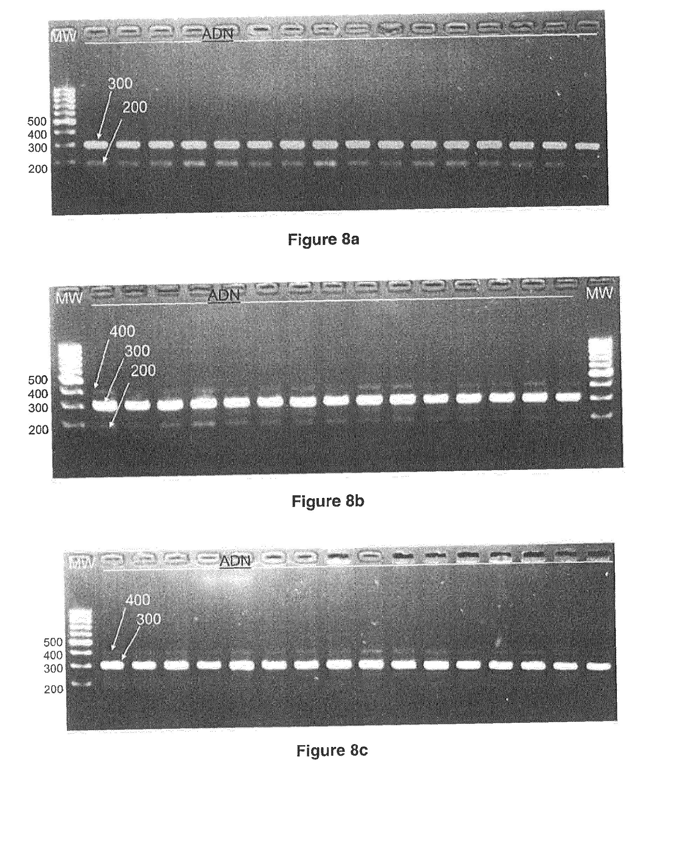

Ce test a été réalisé à température fixe sur le modèle à trois bandes sur un appareil en respectant le schéma de positionnement des 16 points sur le bloc. Sur la figure 8a, la température choisie de 57.6°C précède la température seuil et l'on observe seulement 2 bandes (200 et 300).

Sur la figure 8b, la température choisie de 58.3°C (entre 58,1 et 58,6°C) est la température seuil et l'on observe bien 3 bandes (200, 300, et 400).

Sur la figure 8c, la température choisie de 59°C est la température juste après le seuil et l'on observe seulement 2 bandes (300 et 400).

**Exemple 3 : Description d'un autre test de calibration biologique [Stratégie 3]**

**1. La matrice**

Matrice commerciale : **Gateway® pDEST™14 Vector** d'Invitrogen

Référence catalogue : 11801-016

Plasmide circulaire de 6422pb dosé à 6μg et de séquence connue.

**2. Les amorces du témoin interne positif qui valide le fonctionnement de la réaction PCR quelle que soit la température utilisée entre 50 et 65°C**

La cible est la séquence du gène AMP (ampicilline) du vecteur pDEST14 (taille du gène = 861 pb)

La taille de l'amplicon obtenu est de 78pb.

Les oligos dessinés sur la séquence du gène AMP utilisés sont les suivants :

Code oligo <u>AMPF01</u> : 5' GA TAA ATC TGG AGC CGG TGA 3' (SEQ ID N° 9) avec un simple déssalage au moment de la production de l'oligo par le fournisseur

Code oligo <u>AMPR01</u> : 5' GA TAC GGG AGG GCT TAC CAT 3' (SEQ ID N° 10) avec un simple déssalage au moment de la production de l'oligo par le fournisseur

**3. Les amorces du test biologique qui permettent de détecter un écart de température de 0.5°C au moment de l'étape d'hybridation des amorces sur la matrice**

La cible est la séquence du promoteur pBR322 du vecteur pDEST14 (taille du promoteur = 674pb)

La taille de l'amplicon obtenu lorsque les amorces ci-dessous accrochent est de 198pb

Code oligo <u>pBR322F01</u> : 5' CC GGA TCA AGA GCT ACC AAC 3' (SEQ ID N° 11)avec un simple déssalage au moment de la production de l'oligo par le fournisseur

Code oligo <u>pBR322R03</u> : 5' CA ACC CGG TAA GAC ACG ACC 3' (SEQ ID N° 12) avec une purification de type HPLC au moment de la production de l'oligo par le fournisseur

L'amorce pBR322R03 possède un mismatch sur la dernière base de l'oligo en position 3'. Ce mismatch permet une instabilité de l'accrochage de cette amorce sur la séquence cible en fonction des conditions chimiques et de températures de la réaction PCR.

Le procédé de ce test consiste à déterminer sur le thermocycleur la température seuil (Ts) à partir de laquelle l'amplicon du promoteur pBR322 ne donne aucun signal d'amplification sur gel d'agarose. Ce test est sensible à 0.5°C près.

L'utilisateur devra donc réaliser des essais de gradient de température pour l'étape d'accrochage des amorces pour déterminer cette température seuil limite. Ensuite, il réalisera un test PCR à trois températures fixes d'hybridation des amorces :

- température 1 : Ts - 1 °C
- température 2 : Ts - 0.5°C
- température 3 : Ts

Préférentiellement, pour l'étape de détermination de la température seuil, on met en oeuvre le thermocycleur de manière à générer un gradient de température d'hybridation entre les puits successifs, afin de réaliser, avec le milieu réactionnel décrit ci-dessous, des réactions d'amplification simultanées avec une gamme de températures d'hybridation croissante pour des puits successifs distincts, ladite gamme de températures d'hybridation couvrant au moins la partie médiane de la gamme de températures d'hybridation optimale couverte par l'ensemble des deux paires d'amorces nucléiques utilisées, c'est à dire si possible dans une gamme de températures d'hybridation programmées dans le thermocycleur allant de 58°C à 64°C.

Préférentiellement, on programme l'appareil thermocycleur pour PCR pour réaliser un gradient de températures d'hybridation dont la borne inférieure est d'au moins 55°C, ce qui inclut au moins 55°C, 56°C, 57°C, 58°C, 59°C, 60°C, 61 °C, 62°C, 63°C et 64°C.

Préférentiellement, on programme l'appareil thermocycleur pour PCR pour réaliser un gradient de températures d'hybridation dont la borne supérieure est d'au plus, 70°C, ce qui inclut au plus 65°C, 64°C, 63°C, 62°C et 61 °C.

Par exemple, le thermocycleur peut être programmé pour réaliser un gradient de températures d'hybridation allant de 58°C à 64°C.

Préférentiellement, la température de dénaturation programmée dans le thermocycleur est de 95°C.

Préférentiellement, la température d'élongation programmée dans le thermocycleur est de 72°C.

La valeur de « température seuil », pour l'appareil thermocycleur pour PCR testé, est la température d'hybridation mesurée par l'appareil thermocycleur des puits pour lesquels est observée l'absence d'amplification de l'ADN cible pBR322 avec la paire d'amorces pBR322F01 et pBR322R03.

Préférentiellement, pour mettre en oeuvre le procédé de réglage biologique de thermorégulation d'un thermocycleur PCR, on utilise les paires d'amorces suivantes :

(vii)une paire d'amorces de séquences SEQ ID N°9 et SEQ ID N°10, ayant une température optimale d'hybridation de 60°C,

(viii) une paire d'amorces de séquences SEQ ID N°11 et SEQ ID N°12, ayant une température limite d'hybridation inférieure de 0.5°C à la température seuil (Ts) à partir de laquelle l'amplicon du promoteur pBR322 ne donne aucun signal d'amplification sur gel d'agarose,

4. Les conditions du test

*MIX PCR*

|  | 1 réaction (en µL) | concentration finale |
|---|---|---|
| $H_2O$up | 7,6 |  |
| $MgCl_2$ Promega (25mM) | 1,2 | 1,5mM |
| Tampon 5X Green Promega | 4 | 1X |
| pBR322F01 (10µM) | 0,62 | 0,312µM |
| pBR322R03 (10µM) | 0,62 | 0,312µM |
| AMPF01 (10µM) | 1,25 | 0,625µM |
| AMPR01 (10µM) | 1,25 | 0,625µM |
| dNTP (25mM) | 0,25 | 0,31mM |
| GoTaq Flexi Promega 5U/µL | 0,2 | 1 unité soit 0,0625U/µL |
| volume mix | **17** |  |
| volume ADN (1pg/µL) | **3** |  |
| volume final | 20 |  |

*Programmes PCR*

| Température | Etape | Temps | Nb Cycles |
|---|---|---|---|
| 95°C | Dénaturation initiale | 2 min |  |
| 95°C | Dénaturation | 1min | 28 |
| Ts -1°C et Ts -0.5°C et Ts | Hybridation Primers | 1min |  |
| 72°C | Elongation | 20s |  |
| 72°C | Elongation finale | 5 min |  |
| 15°C | Conservation | forever |  |

Ts : la température seuil à partir de laquelle l'amplicon du promoteur pBR322 ne donne aucun signal d'amplification sur gel d'agarose

*Séparation sur agarose*

■ Dépôt : 10µl du produit PCR contenant déjà le tampon de charge vert

■ TAE 0,5X
■ Cuve BioRad
■ Tension : 80V (soit ~5.5 V.cm$^{-1}$)
■ Durée : 1 h50
■ Gel agarose 3%

## Résultats

Disposition des échantillons dans une plaque de 96 puits, selon le plan suivant :

Puits noirs : 20µl mix PCR + ADN matrice, aux positions respectives A1, A5, A9, A12, B8, C3, C6, C11, E5, E8, F3, F1 0, G8, H1, H5, H12.
Puits blancs : 20µl H2Oup, aux positions restantes des rangées et colonnes de la plaque 96 puits.

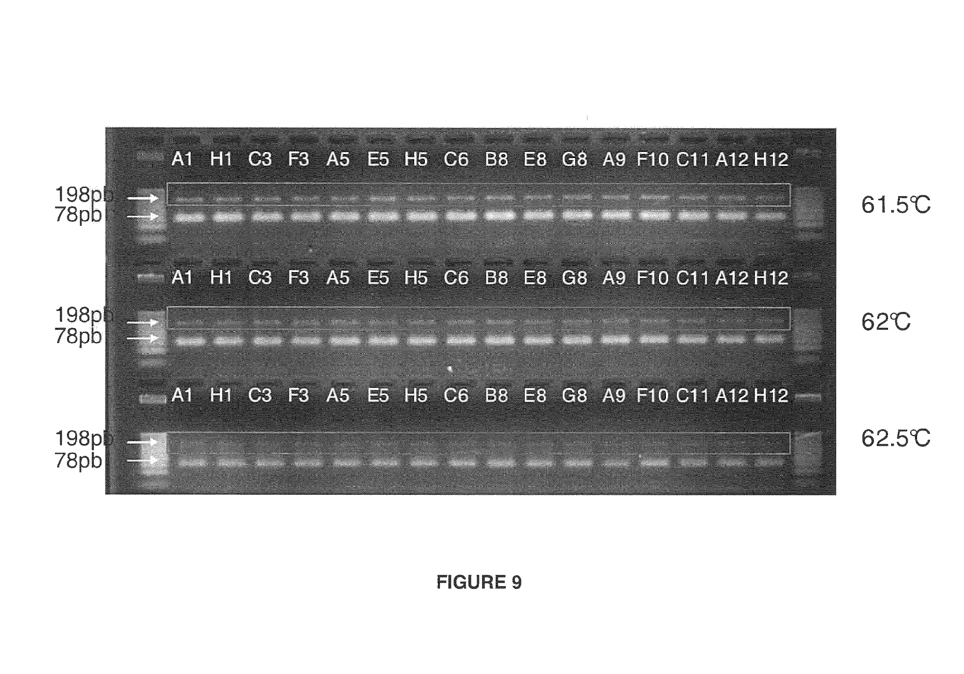

Les résultats sont illustrés sur la figure 9.
Les résultats montrent que les amorces du témoin interne positif (SEQ ID N˚9 et 10) permettent la réalisation de la réaction d'amplification PCR, dans la gamme de températures de 50˚C à 65˚C.
Les résultats montrent également que les amorces du test biologique (SEQ ID N˚ 11 et 12) permettent de détecter un écart de température de 0,5˚C, par rapport à la température désirée.

**Revendications**

1. Procédé pour tester la justesse et la fidélité thermique d'une enceinte thermiquement contrôlée d'un thermocycleur pour PCR, ladite enceinte thermiquement contrôlée comprenant une pluralité d'emplacements pour tubes de mélange réactionnel, ledit procédé comprenant les étapes suivantes :

   a) disposer un ensemble de capteurs de mesure de température dans des emplacements pour tube de mélange réactionnel choisis dans ladite enceinte thermiquement contrôlée, lesdits capteurs de mesure de température comprenant :

   (i) un capillaire ou tube adapté aux emplacements prévus dans le carrousel,
   (ii) un volume choisi de liquide emplissant au moins partiellement ledit tube réactionnel
   (iii) une sonde thermique au moins partiellement immergée dans ledit liquide, et
   (iv) au moins un moyen de liaison de ladite sonde thermique à un moyen de réception d'un signal généré par ladite sonde thermique,
   lesdits emplacements dans lesquels sont disposés les capteurs de mesure de température étant répartis dans l'enceinte thermiquement contrôlée par air pulsé aux emplacements prévus pour les tubes ou capillaires de PCR de manière qu'au moins la moitié ou qu'au plus 20 de ces emplacements soient occupés par des capteurs de mesure, et

   b) démarrer le thermocycleur, dans des conditions normales d'utilisation (c'est-à-dire, couvercle chauffant fermé et actif), pour au moins un cycle programmé avec des valeurs de consigne de température pour une réaction PCR, et
   c) simultanément à l'étape b), mesurer les valeurs de température avec chacun des capteurs de mesure, à une pluralité d'instants donnés.

2. Procédé selon la revendication 1, **caractérisé en ce qu'**à l'étape a) sont disposés au moins 16, 18, 18 et 20 capteurs de mesure de la température pour des enceintes thermiquement contrôlées par air pulsé comportant respectivement des carrousels de 32, 36, 72 et 99 puits.

3. Procédé selon l'une des revendications 1 ou 2, **caractérisé en ce que** les coordonnées de positionnement des capteurs de mesure dans les enceintes thermiquement contrôlées comportant des carrousels de 32, 36, 72 ou 99 puits numérotés respectivement de 1 à 32, de 1 à 36, de 1 à 72 ou de 1 à 99 de manière circulaire, sont les suivantes :

- 32 puits : 1, 3, 5, 7, 9, 11, 13, 15, 17, 19, 21, 23, 25, 27, 29, 31 (un puits sur deux) ;
- 36 puits : 1, 3, 5, 7, 9, 11, 13, 15, 17, 19, 21, 23, 25, 27, 29, 31, 33, 35 (un puits sur deux) ;
- 72 puits : 1, 5, 9, 13, 17, 21, 25, 29, 33, 37, 41, 45, 49, 53, 57, 61, 65, 69 (un puits sur 4) ;
- 99 puits : 1, 5, 10, 15, 20, 25, 30, 35, 40, 45, 50, 55, 60, 65, 70, 75, 80, 85, 90, 95 (un puits sur 5).

**4.** Procédé selon l'une des revendications 1 à 3, **caractérisé en ce que** ledit capteur de mesure de température comprend une sonde à thermocouple de type E, J, K, N, T, R, S, B ou C ou une sonde à résistance de type PT100.

**5.** Procédé selon l'une des revendications 1 à 4, **caractérisé en ce qu'**un gainage complémentaire puisse être ajouté audit capteur de mesure de température, les matières suivantes étant notamment, sinon exclusivement, concernées par l'application : polypropylène, polyéthylène, PTFE, silicone.

**6.** Procédé selon l'une des revendications 1 à 5, **caractérisé en ce que** le moyen de liaison du capteur de mesure consiste en un câble électrique d'un diamètre inférieur à 2 millimètres.

**7.** Procédé selon l'une quelconque des revendications 1 à 6, **caractérisé en ce qu'**il comprend en outre une étape d) de détermination de défauts dans la justesse et la fidélité thermique de l'enceinte thermiquement contrôlée.

**8.** Procédé pour tester la justesse et la fidélité thermique de thermocycleur pour PCR, comprenant les étapes suivantes :

a) réaliser un procédé de test de calibration physique selon l'une des revendications 1 à 7, et
b) réaliser un test de calibration biologique, à l'aide d'une pluralité de paires d'amorces ayant chacune une température optimale d'hybridation connue et distincte.

**9.** Capteur de mesure de la température d'un puits d'une enceinte thermiquement contrôlée pour PCR comprenant :

(i) un tube réactionnel adapté au type de carrousel de l'enceinte thermique testée,
(ii) un volume choisi de liquide emplissant au moins partiellement ledit tube réactionnel,
(iii) une sonde thermique au moins partiellement immergée dans ledit liquide, et
(iv) au moins un moyen de liaison de ladite sonde thermique à un moyen de réception d'un signal généré par ladite sonde thermique, ledit moyen de liaison consistant en un câble de diamètre réduit, permettant son passage entre le bord supérieur du tube réactionnel et le bord du couvercle en position fermée.

**10.** Procédé de réglage biologique de la justesse et la fidélité thermique d'une enceinte thermiquement contrôlée d'un appareil thermocycleur pour PCR, comprenant les étapes suivantes :

a) déterminer, pour ledit appareil thermocycleur, une température seuil (Ts) de réglage, ladite température seuil (Ts) de réglage étant la valeur de température commune où sont vérifiées les conditions (i) et (ii) ci-après :

(i) la température d'hybridation mesurée par le thermocycleur, à laquelle est amplifié un ADN plasmidique circulaire (« ADN cible ») par la paire d'amorces de séquences SEQ ID N˚9 et SEQ ID N˚10, en présence d'une ADN polymérase thermorésistante, et
(ii) la température limite d'hybridation mesurée par le thermocycleur, à laquelle n'est pas amplifié un ADN plasmidique circulaire (« ADN cible ») par la paire d'amorces de séquences SEQ ID N˚11 et SEQ ID N˚12, en présence d'une ADN polymérase thermorésistante, et

b) tester la justesse et la fidélité thermique de l'enceinte thermiquement contrôlée dudit appareil thermocycleur, en réalisant les étapes suivantes :

b1) on réalise trois réactions PCR, à des températures d'hybridation programmée respectivement égales à (1) la température seuil (Ts) déterminée à l'étape a), (2) une température inférieure de 0,5˚C (Ts-0.5˚C) à la température seuil (Ts) déterminée à l'étape a), et (3) une température inférieure de 1 ˚C (Ts-1 ˚C) à la température seuil déterminée à l'étape a),
b2) on vérifie que, (1) auxdites températures (Ts-0.5˚C) et (Ts-1 ˚C), on amplifie ledit ADN cible avec chacune des deux paires d'amorces SEQ ID N˚9-10 et SEQ ID N˚11-12 et (2) à ladite température seuil (Ts) déterminée à l'étape a), on amplifie ledit ADN cible avec uniquement la paire d'amorces de séquences SEQ ID N˚9 et SEQ ID N˚10.

12

15

1

14

11

13

**Fig. 1A**

12

**Fig. 1B**

15

11

**Fig. 1**

Figure 1C

Figure 1D

Figure 1E

Fig. 2

Figure 3A

Figure 3B

Figure 3C

EP 2 522 428 A1

Figure 3D

Figure 3E

Figure 3F

Figure 3G

FIGURE 3H

EP 2 522 428 A1

FIGURE 3I

Figure 3J

FIGURE 3K

Figure 3L

EP 2 522 428 A1

Fig. 4

41

EP 2 522 428 A1

Figure 5A1

Figure 5A2

Figure 5A3

EP 2 522 428 A1

Figure 5B1

EP 2 522 428 A1

Figure 5B2

Figure 5B3

Figure 5C1

Figure 5C2

Figure 5C3

Figure 6a

Figure 6b

Figure 7a

Figure 7b

Figure 8a

Figure 8b

Figure 8c

FIGURE 9

Europäisches
Patentamt

European
Patent Office

Office européen
des brevets

**RAPPORT DE RECHERCHE EUROPEENNE**

Numéro de la demande

EP 12 16 9223

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | KIM YOUNG HO ET AL: "Performance evaluation of thermal cyclers for PCR in a rapid cycling condition", BIOTECHNIQUES, vol. 44, no. 4, avril 2008 (2008-04), pages 495-496,498,50, XP002557398, ISSN: 0736-6205 | 9 | INV. B01L7/00 C12Q1/68 |
| Y | * abrégé; figure 1 * | 1-8 | |
| X,D | YANG I ET AL: "Use of multiplex polymerase chain reactions to indicate the accuracy of the annealing temperature of thermal cycling", ANALYTICAL BIOCHEMISTRY, ACADEMIC PRESS INC, NEW YORK, vol. 338, no. 2, 15 mars 2005 (2005-03-15), pages 192-200, XP004770819, ISSN: 0003-2697 | 9 | |
| Y | * le document en entier * | 1-8 | |
| X | SCHODER DAGMAR ET AL: "Physical characteristics of six new thermocyclers.", CLINICAL CHEMISTRY, vol. 49, no. 6, juin 2003 (2003-06), pages 960-963, XP002557473, ISSN: 0009-9147 | 9 | DOMAINES TECHNIQUES RECHERCHES (IPC)<br><br>B01L |
| Y | * le document en entier * | 1-8 | |
| X,D | SCHODER D ET AL: "Novel approach for assessing performance of PCR cyclers used for diagnostic testing", JOURNAL OF CLINICAL MICROBIOLOGY 200506 US, vol. 43, no. 6, juin 2005 (2005-06), pages 2724-2728, XP002557399, ISSN: 0095-1137 | 9 | |
| Y | * le document en entier * | 1-8 | |

-/--

Le présent rapport a été établi pour toutes les revendications

| Lieu de la recherche | Date d'achèvement de la recherche | Examinateur |
|---|---|---|
| Munich | 9 octobre 2012 | Schmitt-Humbert, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**Europäisches Patentamt**
**European Patent Office**
**Office européen des brevets**

## RAPPORT DE RECHERCHE EUROPEENNE

Numéro de la demande

EP 12 16 9223

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2002/072112 A1 (ATWOOD JOHN GIRDNER [US] ET AL ATWOOD JOHN GIRDNER [US]) 13 juin 2002 (2002-06-13) * le document en entier * ----- | 1-8 | |
| X | "ISO/TS 20836. Microbiology of food and animal feeding stuff. Polymerase chain reaction (PCR) for the detection of food-borne pathogens. Performance testing for thermal cyclers = Reaction de polymérisation en chaîne (PCR) pour la recherche de micro-organismes pathogènes dans les aliments - Essais de", INTERNATIONAL STANDARD - ISO, ZUERICH, CH, vol. 20836, 1 août 2006 (2006-08-01), page 19pp, XP009134418, * le document en entier, en particulier les annexes A et B * ----- | 1-8 | |
| A | WITTWER C T ET AL: "THE LIGHTCYCLERTM: A MICROVOLUME MULTISAMPLE FLUORIMETER WITH RAPID TEMEPRATURE CONTROL", BIOTECHNIQUES, INFORMA HEALTHCARE, US, vol. 22, no. 1, 1 janvier 1997 (1997-01-01), pages 176-181, XP001094113, ISSN: 0736-6205 * le document en entier * ----- -/-- | 1-8 | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| Munich | 9 octobre 2012 | Schmitt-Humbert, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...................................................................................................
& : membre de la même famille, document correspondant

EPO FORM 1503 03.82 (P04C02)

**EP 2 522 428 A1**

Europäisches
Patentamt

European
Patent Office

Office européen
des brevets

# RAPPORT DE RECHERCHE EUROPEENNE

Numéro de la demande

EP 12 16 9223

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CORBETT LIFE SCIENCE: "Rotor-Gene 6000 real time rotary analyzer", INTERNET CITATION, 2007, XP002469624, Extrait de l'Internet: URL:http://www.corbettlifescience.net/publ ic/marketing/documents/rotor-gene_a4_72dpi .pdf [extrait le 2008-02-19] * le document en entier * ----- | 1-8 | |
| A,P | GROENLUND H. A. ET AL: "The use of infrared thermography as a novel approach for real -time validation of PCR thermocyclers", FOOD ANAL. METHODS DOI: 10.1007/S12161-009-9103-2, 15 septembre 2009 (2009-09-15), XP002557400, * le document en entier * ----- | 1-8,10 | |
| X,P | ANONYMOUS: "Biologie moléculaire-Comment déterminer les performances des thermocycleurs?", INTERNET CITATION, février 2009 (2009-02), pages 1-2, XP002586792, Extrait de l'Internet: URL:http://www.lne.fr/fr/formation/fiches_ journees_techniques/2009/jt902-biologie-mo leculaire.pdf [extrait le 2010-06-09] * le document en entier * ----- | 1-10 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 9 octobre 2012 | Schmitt-Humbert, C |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
....................................................................
& : membre de la même famille, document correspondant

EPO FORM 1503 03.82 (P04C02)

59

**EP 2 522 428 A1**

ANNEXE AU RAPPORT DE RECHERCHE EUROPEENNE
RELATIF A LA DEMANDE DE BREVET EUROPEEN NO.

EP 12 16 9223

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2012

| Document brevet cité au rapport de recherche | Date de publication | Membre(s) de la famille de brevet(s) | Date de publication |
|---|---|---|---|
| US 2002072112 A1 | 13-06-2002 | US 2002072112 A1 | 13-06-2002 |
| | | US 2004086927 A1 | 06-05-2004 |
| | | US 2005084957 A1 | 21-04-2005 |
| | | US 2006269641 A1 | 30-11-2006 |
| | | US 2006286659 A1 | 21-12-2006 |
| | | US 2008299651 A1 | 04-12-2008 |
| | | US 2009155765 A1 | 18-06-2009 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **KIM et al.** *BioTechniques,* 2008, vol. 44 (4), 495-505

- **YANG et al.** Use of multiplex polymerase chain reactions to indicate the accuracy of the annealing temperature of thermal cycling. *Analytical Biochemistry,* 2005, vol. 338, 192-200